# EUROPÄISCHE PATENTSCHRIFT

(11) **EP 4 196 099 B1**
(45) Veröffentlichungstag und Bekanntmachung des Hinweises auf die Patenterteilung: **12.02.2025**
(21) Anmeldenummer: 21773526.5
(22) Anmeldetag: 06.09.2021
(51) Int. Cl.: A61K 31/00, A61K 31/12, A61K 31/136, A61K 31/255, A61K 31/7012, A61K 45/06, A61P 31/12, A61P 31/14, A61P 31/18

(54) **DIPHENYLDERIVATE FÜR DIE VERWENDUNG IN DER THERAPIE VON VIRUSERKRANKUNGEN, INSBESONDERE CORONA-INFEKTIONEN, INSBESONDERE COVID-19**
DIPHENYL DERIVATIVES FOR THE USE IN THE THERAPY OF VIRAL DISEASES, IN PARTICULAR CORONA INFECTIONS, IN PARTICULAR COVID-19
DÉRIVÉS DIPHÉNYLIQUES POUR L'UTILISATION DANS LA THÉRAPIE DE MALADIES VIRALES, EN PARTICULIER D'INFECTIONS CORONA, EN PARTICULIER DE COVID-19

(30) Priorität: 25.09.2020 DE 102020125125; 01.10.2020 DE 102020125641
(43) Veröffentlichungstag der Anmeldung: 21.06.2023
(73) Patentinhaber: Creative Therapeutics GmbH, 42113 Wuppertal (DE)
(72) Erfinder: ZEILER, Hans-Joachim, 42113 Wuppertal (DE)
(74) Vertreter: Strehlke, Ingo Kurt
(86) Internationale Anmeldenummer: PCT/EP2021/074443
(87) Internationale Veröffentlichungsnummer: WO 2022/063549

(56) Entgegenhaltungen:
- WO-A1-2012/122969
- WO-A1-2012/130850
- WO-A2-02/48147
- WO-A2-2011/000566
- DE-A1- 102015 008 631
- MANFRONI GIUSEPPE ET AL: "Inhibition of Subgenomic Hepatitis C Virus RNA Replication by Acridone Derivatives: Identification of an NS3 Helicase Inhibitor", JOURNAL OF MEDICINAL CHEMISTRY, vol. 52, no. 10, 28 May 2009 (2009-05-28), US, pages 3354 - 3365, XP055871451, ISSN: 0022-2623, DOI: 10.1021/jm801608u
- LUYING CHEN ET AL: "Isolation, identification and antiviral activities of metabolites of calycosin-7----glucopyranoside", JOURNAL OF PHARMACEUTICAL AND BIOMEDICAL ANALYSIS, ELSEVIER B.V, AMSTERDAM, NL, vol. 56, no. 2, 25 May 2011 (2011-05-25), pages 382 - 389, XP028098552, ISSN: 0731-7085, [retrieved on 20110601], DOI: 10.1016/J.JPBA.2011.05.033
- WYATT P G ET AL: "Benzophenone derivatives: a novel series of potent and selective inhibitors of human immunodeficiency virus type 1 reverse transcriptase", JOURNAL OF MEDICINAL CHEMISTRY, AMERICAN CHEMICAL SOCIETY, US, vol. 38, no. 10, 1 January 1995 (1995-01-01), pages 1657 - 1665, XP002221133, ISSN: 0022-2623, DOI: 10.1021/JM00010A010
- NEAMATI N ET AL: "DEPSIDES AND DEPSIDONES AS INHIBITORS OF HIV-1 INTEGRASE: DISCOVERYOF NOVEL INHIBITORS THROUGH 3D DATABASE SEARCHING", JOURNAL OF MEDICINAL CHEMISTRY, AMERICAN CHEMICAL SOCIETY, US, vol. 40, no. 6, 1 January 1997 (1997-01-01), pages 942 - 951, XP002924579, ISSN: 0022-2623, DOI: 10.1021/JM960759E
- MANFRONI GIUSEPPE ET AL: "Inhibition of Subgenomic Hepatitis C Virus RNA Replication by Acridone Derivatives: Identification of an NS3 Helicase Inhibitor", JOURNAL OF MEDICINAL CHEMISTRY, vol. 52, no. 10, 28 May 2009 (2009-05-28), US, pages 3354 - 3365, XP055871451, ISSN: 0022-2623, DOI: 10.1021/jm801608u

## Beschreibung

Die vorliegende Erfindung betrifft das Gebiet der Therapie von viralen Infektionen (Virusinfektionen), insbesondere Infektionen mit Corona-Viren, insbesondere COVID-19. Insbesondere betrifft die vorliegende Erfindung Wirkstoffe und Arzneimittel zur Verwendung für eine solche Therapie.

Insbesondere betrifft die vorliegende Erfindung Wirkstoffe und Arzneimittel zur Verwendung bei der prophylaktischen und/oder therapeutischen Behandlung von Viruserkrankungen, insbesondere Corona-Infektionen, insbesondere COVID-19, bzw. die Verwendung von Wirkstoffen und Arzneimitteln zur prophylaktischen und/oder therapeutischen Behandlung von Viruserkrankungen, insbesondere Corona-Infektionen, insbesondere COVID-19. Im Rahmen der vorliegenden Erfindung kommen dabei spezielle Wirkstoffe auf Basis von Diphenylderivaten bzw. diese speziellen Wirkstoffe enthaltende Arzneimittel zum Einsatz, wie sie in der nachfolgenden Beschreibung und in den Patentansprüchen zu der vorliegenden Erfindung definiert sind.

Coronaviridae ist eine Virusfamilie innerhalb der Ordnung Nidovirales. Die Viren innerhalb dieser Virusfamilie werden fachumgangssprachlich auch Coronaviren genannt und gehören zu den RNA-Viren mit den größten Genomen. Die ersten Corona-viren wurden bereits Mitte der 1960er Jahre entdeckt und beschrieben. Die im elektronenmikroskopischen Bild grob kugelförmigen Viren fallen durch einen Kranz blütenblattartiger Fortsätze auf, die an eine Sonnenkorona erinnern, welche dieser Virusfamilie ihren Namen gegeben hat.

Vertreter der Virenfamilie der Coronaviridae verursachen bei allen vier Klassen der Landwirbeltiere (d. h. Säugetiere, Vögel, Reptilien und Amphibien) sehr unterschiedliche Erkrankungen. Sie sind genetisch hochvariabel und können so auch mehrere Arten von Wirten infizieren. Beim Menschen sind sieben Arten von Coronaviren als Erreger von leichten respiratorischen Infektionen (insbesondere Erkältungen bzw. grippalen Infekten) bis hin zum sogenannten schweren akuten Atemwegssyndrom (SARS bzw. *Severe Acute Respiratory Syndrome*) von Bedeutung.

Unter den menschlichen Coronaviren besonders bekannt geworden sind die folgenden Coronaviren: SARS-CoV-1 *(Severe Acute Respiratory Syndrome Coronavirus-*1), MERS-CoV *(Middle East Respiratory Syndrome Coronavirus)* und SARS-CoV-2 *(Severe Acute Respiratory Syndrome Coronavirus 2* bzw. COVID-19). Diese Coronaviren sind die Auslöser der SARS-Pandemie 2002/2003, der MERS-Epidemie 2012 bzw. der COVID-19-Pandemie ab 2019.

COVID-19 (d.h. die Abkürzung für Englisch *Coronavirus-Disease 2019* oder Deutsch Coronavirus-Krankheit-2019, umgangssprachlich auch als Coronavirus-Erkrankung oder dergleichen bezeichnet) ist eine Infektionskrankheit, in deren Folge es zu einer Infektion mit dem neuartigen Coronavirus SARS-CoV-2 kommt. Die primär die Atemwege befallende Erkrankung wurde erstmals Ende des Jahres 2019 in Wuhan/China beschrieben, entwickelte sich dann im Januar 2020 zunächst in der Volksrepublik China zu einer Epidemie und breitete sich schließlich weltweit zur COVID-19-Pandemie aus.

Die Ansteckung bei COVID-19 erfolgt in der Regel durch Tröpfchenübertragung. Die Inkubationszeit von COVID-19 beträgt durchschnittlich fünf bis sechs Tage, wobei zwischen Ansteckung und dem Auftreten erster Symptome bisweilen aber auch bis zu zwei Wochen vergehen können und vereinzelt erste Symptome schon innerhalb von 24 Stunden nach Ansteckung mit SARS-CoV-2 auftreten können. Die häufigsten Symptome sind Fieber, trockener Husten und Müdigkeit, weniger häufig sind dagegen Muskelschmerzen, eine verstopfte Nase, Kopfschmerzen, Bindehautentzündung, Halsschmerzen, Durchfall, Geschmacks- oder Geruchsverlust oder Hautausschlag oder Verfärbung von Fingern oder Zehen zu beobachten. Infizierte ohne Symptome können trotzdem potentielle Überträger des Coronavirus sein. Bei einem leichten Krankheitsverlauf klingen die Symptome im Allgemeinen innerhalb von zwei Wochen ab. Verläuft COVID-19 schwerer, kann die Rekonvaleszenz drei bis sechs Wochen oder sogar noch länger andauern. Bei rund 81 % der registrierten Infektionen ist ein leichter Krankheitsverlauf mit Fieber oder einer leichten Lungenentzündung zu beobachten; bei etwa 14 % der Fälle ist jedoch der Verlauf schwerer, und in etwa 5 % der Fälle ist der Verlauf sogar derart schwer, dass eine intensivstationäre Betreuung der Patienten erfolgen muss.

COVID-19 ist zurzeit Gegenstand intensiver Forschung. Effiziente und spezifische kausale Therapien oder Impfungen stehen zurzeit noch nicht zur Verfügung.

Das COVID-19 auslösende Virus SARS-CoV-2 dringt - wie auch SARS-CoV-1 bei SARS - typischerweise über eine Bindung an das in der Zellmembran verankerte Enzym ACE2 (Angiotensin-konvertierendes Enzym 2) in die menschliche Zelle ein, wobei das virale Spike-Protein mit ACE2 interagiert. Für diesen Prozess ist die Mitwirkung der Serinprotease TMPRSS2 (Transmembrane Serinprotease 2) erforderlich. Im Versuch mit HeLa-Zellen, welche ACE2 des Menschen, der Chinesischen Hufeisennase (Rhinolophus sinicus), einer Schleichkatzenart, des Hausschweins und der Maus exprimieren, konnte SARS-CoV-2 das jeweilige ACE2-Protein als Rezeptor nutzen, um in die Zelle einzudringen; nur bei dem Maus-ACE2-Enzym gelang dies nicht - ebenso wenig bei HeLa-Zellen, welche kein ACE2 bilden. An Rezeptoren, welche von anderen Coronaviren genutzt werden, findet dagegen keine Bindung von SARS-CoV-2 statt.

Neben dem Weg über ACE2 wurden bei T-Lymphozyten, welche kein oder wenig ACE2 auf ihrer Oberfläche tragen, ein alternativer Eindringweg experimentell nachgewiesen: Das Virus dringt bei diesen Zellen über eine durch das Spike-Protein vermittelte Verschmelzung der Virusmembran mit der Lymphozytenzellmembran ein.

Eine reverse Suche in einer humanen Gendatenbank (*Human Cell Atlas* oder kurz *HCA*) nach Zelltyp und Geweben, bei welchen neben ACE2 auch TMPRSS₂ auf Membranoberflächen vorhanden ist, zeigt, dass in der Nasenschleimhaut, insbesondere den Becherzellen, aber auch den Flimmerepithelen, die höchsten Konzentrationen dieser beiden Proteine auftreten, weshalb diese Zellen als Eintrittspforte für SARS-CoV-2 angesehen und auch als Reservoir vermutet werden. Die Proteine werden ebenso in den Hornhaut-Zellen des Auges, in der Darmschleimhaut sowie im Herzen in Perizyten der Blutkapillaren, Herzmuskelzellen und Fibroblasten gebildet. Dabei bleibt die erste Phase des Befalls im Nasenrachen nahezu symptomfrei, wohingegen bei Übergang in eine schwere Verlaufsform überwiegend die Lunge angegriffen wird, da ein Großteil der ACE-2-exprimierenden Zellen des Menschen in den Typ-II-Pneumozyten der Lunge vorkommt. Als weitere Gründe für die besondere Anfälligkeit der Lunge wird ihre große Oberfläche angegeben; außerdem verfügen die ACE-2-exprimierenden Pneumozyt-Typ-II-Zellen über diverse Gene, welche die Replikation und Transmission von SARS-CoV-2 begünstigen.

Bei Untersuchungen an kryokonservierten Lungengewebsproben von Nichtinfizierten kann auch gezeigt werden, dass Lungengewebe kaum ACE2 sowie die Transmembranprotease TMPRSS2 ausbildet, die Pneumozyten Typ II in der Lunge hingegen vermehrt. Diese Vorläuferzellen sind bei Männern und in fortgeschrittenem Alter tendenziell vermehrt nachzuweisen. Neben unterschiedlichen ACE2-Werten bei Männern und Frauen wird eine Ursache für die unterschiedliche schwere der Erkrankung im geschlechtsspezifischen Hormonhaushalt vermutet: Östrogen fördert eine Immunantwort, wohingegen Testosteron diese unterdrückt.

Neuere Erkenntnisse zeigen auch, dass im Lungenepithel und benachbarten Gewebezellen die Proprotease Furin coexprimiert wird, was wiederum dem Virus den Zellzutritt vereinfacht, da es am Spike-Protein eine Furin-spezifische Trennstelle aufweist.

Außer in der Lunge wurde ACE2 auch im Dünn- und Dickdarm, in den Atemwegen und in den Nieren nachgewiesen. Eine Vermehrung des Virus in Darmzellen wurde ebenfalls bestätigt.

Was klinische Symptome und laborchemische Krankheitszeichen anbelangt, so ist eine Abgrenzung von anderen Viruserkrankungen, wie etwa Influenza, allein anhand der Symptome schwierig. Nach einer Inkubationszeit von typischerweise fünf bis sechs Tagen, in seltenen Fällen bis zu 14 Tagen, können - wie eingangs beschrieben - Fieber, Muskelschmerzen und trockener Husten auftreten. Häufig manifestiert sich die Krankheit auch mit allgemeinem schwerem Krankheitsgefühl. Im weiteren Verlauf kann sich dann eine schwere Atemnot aufgrund einer Infektion der unteren Atemwege bis zur Lungenentzündung entwickeln. Diese kann mit Brustschmerzen im Sinne einer Pleuritis einhergehen. Etwa 85 % der schwer erkrankten COVID-19-Patienten entwickeln eine Lymphopenie. Die schwer erkrankten Patienten entwickeln häufig zudem eine Hyperzytokinämie (sogenannter Zytokinsturm), wobei dieser Zytokinsturm durch eine Überreaktion des Immunsystems entsteht; diese Überreaktion ist durch einen deutlichen Anstieg von entzündungsrelevanten Zytokinen gekennzeichnet, wie insbesondere Interleukin-6, Interleukin-8, Interleukin-1β und TNF-α. Die verstärkte Freisetzung dieser Zytokine führt zu einer Überproduktion von Immunzellen, vor allem im Lungengewebe.

Die Diagnose von COVID-19 kann insbesondere durch labordiagnostischen Nachweis erfolgen, insbesondere mittels spezifischer Virus- und Antikörpernachweise.

Eine effiziente und spezifische kausale Therapie konnte bislang noch nicht etabliert werden. Das Nukleosidanalogon Remdesivir zeigt in einer vorläufig veröffentlichten randomisierten Studie eine Verkürzung der Krankheitsdauer bei hospitalisierten Patienten. Dieses Medikament ist in der Europäischen Union für COVID-19-Patienten, welche Sauerstoff benötigen, zugelassen und kann laut Leitlinie der DIVI (Deutsche Interdisziplinäre Vereinigung für Intensiv- und Notfallmedizin e.V.) bei schwerkranken Patienten erwogen werden.

Chloroquin und Hydroxychloroquin dagegen zeigen - entgegen ursprünglicher Erwartungen - keine Hinweise auf eine Wirksamkeit.

Tocilizumab, ein monoklonaler Antikörper, welcher unter anderem zur Behandlung verschiedener Formen von rheumatoider Arthritis und dem Zytokin-Freisetzungssyndrom zugelassen ist, hat sich ebenfalls als unwirksam erwiesen.

Antikörperreiches Plasma genesener Patienten dagegen scheint geeignet zu sein, um Akutfälle zu therapieren, kann aber einen Erfolg nur in der Frühphase der Erkrankung belegen.

Weitere Studien belegen, dass Dexamethason die Sterberate bei Patienten an Beatmungsgeräten von 41 % auf 29 % und bei Patienten mit Sauerstoffversorgung von 26 % auf 23 % reduziert. Bei Patienten, welche keine Sauerstoffgabe benötigen, zeigt eine Behandlung mit Dexamethason dagegen keine positive Wirkung. Dexamethason soll eine überschießende Reaktion des Immunsystems, insbesondere den sogenannten Zytokinsturm, vermindern.

Bei schwer erkrankten COVID-19-Patienten wird zudem eine Gabe von niedrigmolekularem Heparin empfohlen, um das Risiko von Thrombosen und Lungenembolien zu senken. Aufgrund des hohen Vorkommens von Lungenembolien und Beinvenenthrombosen in Verbindung mit schweren Verläufen von COVID-19 wird die Gabe stärker gerinnungshemmender Präparate in Erwägung gezogen; diesbezüglich liegen jedoch noch keine ausreichenden Daten über Nutzen und Risiko vor.

Bisher ist auch noch unklar, inwieweit eine durchgemachte Infektion eine Immunität verleiht; bislang wird auf Basis von Erkenntnissen aus Tiermodellen davon ausgegangen, dass eine akute Immunität besteht, wobei jedoch nicht feststeht, wie lang diese andauert. Auch der Forschungsstand zu Spätfolgen und zur Abschätzung des Sterberisikos ist noch ungewiss.

Die WO 2011/000566 A2 bezieht sich auf die dort beschriebenen Verbindungen und pharmazeutischen Zusammensetzungen sowie deren Verwendung zur Herstellung eines Medikaments zur Behandlung, Linderung oder Vorbeugung von Krankheitszuständen, welche durch eine Virusinfektion mit negative-sense ssRNA-Viren verursacht werden.

Die WO 02/48147 A2 betrifft die dort beschriebenen Verbindungen, pharmazeutische Formulierungen, welche diese Verbindungen umfassen, und die Verwendung dieser Verbindungen in der Therapie, wobei insbesondere auf die Prophylaxe und Behandlung von Infektionen mit Herpes-Viren abgestellt wird.

Chen et al., "Isolation, identification and antiviral activities of metabolites of calycosin-7-O-β-D-glucopyranoside", Journal of Pharmaceutical and Biomedical Analysis, Bd. 56, Nr. 2, 25. Mai 2011, Seiten 382 bis 389, betrifft Untersuchungen zu In-vivo- und In-vitro-Metaboliten von Calycosin-7-O-β-D-Glucopyranoside.

Weiterhin betrifft Wyatt P. G. et al., "Benzophenone Derivatives: A Novel Series of Potent and Selective Inhibitors of Human Immunodeficiency Virus Type 1 Reverse Transcriptase", Journal of Medicinal Chemistry, Bd. 38, Nr. 10, 1. Januar 1995, Seiten 1657 bis 1665, Untersuchungen an synthetisierten Benzophenon-Derivaten mit einer diesbezüglichen Bewertung ihrer Eigenschaft als Inhibitoren der HIV-1-Reversen-Transkriptase und des Wachstums von HIV-1 in MT-4-Zellen.

Zudem betrifft Neamati N. et al., "Depsides and Depsidones as Inhibitors of HIV-1 Integrase: Discovery of Novel Inhibitors through 3D Database Searching", Journal of Medicinal Chemistry, Bd. 40, Nr. 6, 1. Januar 1997, Seiten 942 bis 951, Untersuchungen an verschiedenen Flechtensäuren auf Basis von Depsiden bzw. Depsidonen und ihren synthetischen Derivaten im Hinblick auf ihre hemmende Wirkung gegen die HIV-1-Integrase, wobei Pharmakophore identifiziert worden sind, welche mit der Hemmung dieses Enzyms in Zusammenhang stehen.

Außerdem betrifft Manfroni G. et al., "Inhibition of Subgenomic Hepatitis C Virus RNA Replication by Acridone Derivatives: Identification of an NS3 Helicase Inhibitor", Journal of Medicinal Chemistry, Bd. 52, Nr. 10, 28. Mai 2009, Seiten 3354 bis 3365, die Synthese und die Struktur-Aktivitäts-Beziehung (SAR) von Acridonen und Acridon-Fragment-Derivaten, welche auf der Grundlage der selektiven Anti-Hepatitis-C-Virus (HCV)-Aktivität von Acridon 2 entwickelt wurden.

Die WO 2012/122969 A1 betrifft substituierte Diphenylderivate bzw. Triacen-substituierte Diphenylderivate, welche sich als Chemotherapeutika zur Behandlung von Karzinomen bei Menschen und Tieren eignen sollen.

Darüber hinaus betrifft die DE 10 2015 008 631 A1 Benzophenonderivate, welche sich als Wirkstoffe bei der medikamentösen Behandlung von Krebs eignen sollen.

Die WO 2012/130850 A1 betrifft dort beschriebene substituierte verbrückte Diphenylderivate, welche pro Molekül mindestens eine Dialkyltriazenyl- und mindestens eine ausgewählte Oxycarbonsäuregruppe enthalten, deren Salze, Solvate und die Solvate dieser Salze, wobei die Verwendung dieser Derivate zur Behandlung bzw. Prävention von Krankheiten, insbesondere von Krebserkrankungen, wie beispielsweise Krebserkrankungen der Sexualorgane, beschrieben ist.

Im Ergebnis zielen daher die weltweit meisten Maßnahmen auf einer Prävention von COVID-19-Erkrankungen ab, insbesondere durch verstärkte individuelle Hygienemaßnahmen, wie das Tragen eines Mund-Nasen-Schutzes, Handdesinfektion, Flächenreinigung etc., da bislang eine etablierte kausale Therapie zur kurativen Behandlung oder eine Impfung zu Zwecken der Prävention noch nicht existiert.

Vor diesem Hintergrund besteht eine Aufgabe der vorliegenden Erfindung darin, eine effiziente, vorzugsweise kausale, Therapie von Viruserkrankungen bzw. viralen Infektionen (Virusinfektionen), insbesondere Infektionen mit Corona-Viren, insbesondere COVID-19, bereitzustellen.

Insbesondere ist eine Aufgabe der vorliegenden Erfindung darin zu sehen, Wirkstoffe und diese Wirkstoffe enthaltende Arzneimittel aufzufinden bzw. bereitzustellen, welche sich zur Verwendung bei der prophylaktischen und/oder therapeutischen Behandlung von Viruserkrankungen bzw. viralen Infektionen (Virusinfektionen), insbesondere Corona-Infektionen, insbesondere COVID-19, eignen, bevorzugt im Rahmen einer effizienten, insbesondere kausalen, Therapie.

In vollkommen überraschender Weise hat die Anmelderin aber nunmehr herausgefunden, dass spezielle Wirkstoffe auf Basis von Diphenylderivaten, wie sie in der nachfolgenden Beschreibung und in den Patentansprüchen zu der vorliegenden Erfindung näher definiert sind und welche bislang nur für die Therapie von Tumor- bzw. Krebserkrankungen in Betracht gezogen bzw. eingesetzt worden sind, sich unerwartet auch in effizienter Weise für die bevorzugt kausale Therapie von Viruserkrankungen bzw. viralen Infektionen (Virusinfektionen), insbesondere Infektionen mit Corona-Viren, insbesondere COVID-19, eignen.

Zur Lösung der zuvor geschilderten Problemstellung schlägt daher die vorliegende Erfindung - gemäß einem **ersten** Aspekt der vorliegenden Erfindung - Wirkstoffe bzw. Diphenylderivate zur Verwendung bei der prophylaktischen und/oder therapeutischen Behandlung von viralen Infektionen (Virusinfektionen), insbesondere Infektionen mit Corona-Viren, insbesondere COVID-19, gemäß Patentanspruch 1 vor. Jeweils vorteilhafte Weiterbildungen und Ausgestaltungen dieses Erfindungsaspekts sind Gegenstand der betreffenden Unteransprüche (d. h. Patentanprüche 2 und 6).

Vorliegend beschrieben ist zudem die Verwendung der vorgenannten Wirkstoffe bzw. Diphenylderivate zur prophylaktischen und/oder therapeutischen Behandlung von viralen Infektionen (Virusinfektionen), insbesondere Infektionen mit Corona-Viren, insbesondere COVID-19, bzw. die Verwendung der vorgenannten Wirkstoffe bzw. Diphenylderivate zur Herstellung eines Arzneimittels oder Medikaments zur prophylaktischen und/oder therapeutischen Behandlung von viralen Infektionen (Virusinfektionen), insbesondere Infektionen mit Corona-Viren, insbesondere COVID-19. Vorliegend beschrieben ist auch die Verwendung der vorgenannten Wirkstoffe bzw. Diphenylderivate als Antivirusmittel zur prophylaktischen und/oder therapeutischen Behandlung von Viruserkrankungen, insbesondere Corona-Infektionen, insbesondere COVID-19, bzw. zur Herstellung eines Antivirusmittel zur prophylaktischen und/oder therapeutischen Behandlung von Viruserkrankungen, insbesondere Corona-Infektionen, insbesondere COVID-19.

Weiterhin beschrieben ist ein Verfahren zur prophylaktischen und/oder therapeutischen Behandlung von Viruserkrankungen, insbesondere Corona-Infektionen, insbesondere COVID-19.

Ebenfalls Gegenstand der vorliegenden Erfindung - gemäß einem **zweiten** Aspekt der vorliegenden Erfindung - ist ein Arzneimittel oder Medikament zur Verwendung bei der prophylaktischen und/oder therapeutischen Behandlung von Viruserkrankungen, insbesondere Corona-Infektionen, insbesondere COVID-19, insbesondere Antivirusmittel gemäß Patenanspruch 2. Jeweils vorteilhafte Weiterbildungen und Ausgestaltungen dieses Erfindungsaspekts sind Gegenstand des betreffenden Unteranspruchs (d. h. Patenanspruch 6).

Schließlich ist Gegenstand der vorliegenden Erfindung - gemäß einem **dritten** Aspekt der vorliegenden Erfindung - gleichermaßen eine pharmazeutische Kombination zur Verwendung bei der prophylaktischen und/oder therapeutischen Behandlung von Viruserkrankungen, insbesondere Corona-Infektionen, insbesondere COVID-19, gemäß Patentanspruch 4. Jeweils vorteilhafte Weiterbildungen und Ausgestaltungen dieses Erfindungsaspekts sind Gegenstand der betreffenden Unteransprüche (d. h. Patentansprüche 5 und 6).

Es versteht sich von selbst, dass Ausgestaltungen, Ausführungsformen, Vorteile und dergleichen, welche nachfolgend zu Zwecken der Vermeidung von Wiederholungen zu einem Erfindungsaspekt aufgeführt sind, selbstverständlich auch in Bezug auf die übrigen Erfindungsaspekte entsprechend gelten, ohne dass dies einer gesonderten Erwähnung bedarf.

Darüber hinaus verhält es sich im Hinblick auf die nachfolgende Beschreibung der vorliegenden Erfindung auch derart, dass die jeweils im Zusammenhang mit den speziellen Ausgestaltungen, Ausführungsformen, Vorteilen, Beispielen oder dergleichen angeführten Merkmale der vorliegenden Erfindung auch in deren Kombination als offenbart gelten. Somit gelten vorliegend auch übergeordnete Kombinationen einzelner oder mehrerer Merkmale, welche für jeweilige Ausgestaltungen, Ausführungsformen, Anwendungsbeispiele oder dergleichen angeführt sind, als offenbart.

Gegenstand der vorliegenden Erfindung - gemäß einem **ersten** Aspekt der vorliegenden Erfindung - ist somit ein Diphenylderivat zur Verwendung bei der prophylaktischen und/oder therapeutischen Behandlung von Viruserkrankungen, insbesondere Corona-Infektionen, insbesondere COVID-19,
wobei das Diphenylderivat ein Diphenylderivat gemäß der nachstehenden Formel (IV-A1) oder (IV-A2) ist: 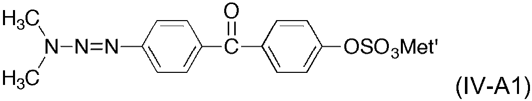 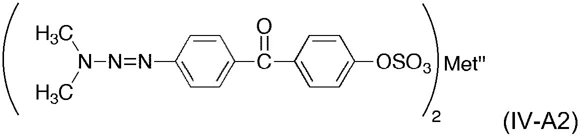 worin:
Met' ein einwertiges Metallion, insbesondere ein Alkalimetallion, vorzugsweise ein Natriumion, bezeichnet und
Met" ein zweiwertiges Metallion, insbesondere ein Erdalkalimetallion, bezeichnet; oder
wobei das Diphenylderivat ein Diphenylderivat gemäß der nachstehenden Formel (IV-A3) ist: 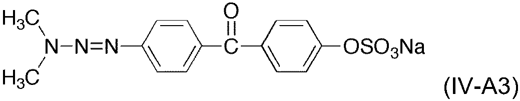 oder
wobei das Diphenylderivat ein Diphenylderivat gemäß der nachstehenden Formel (IV-A) sowie dessen Salze ist: 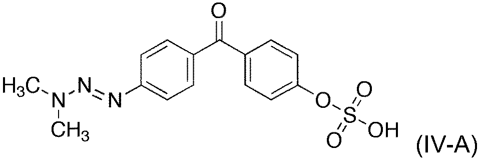 oder
wobei das Diphenylderivat ein Diphenylderivat gemäß den nachstehenden Formeln (IV-B) sowie dessen Salze ist: 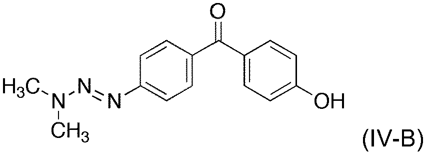 oder
wobei das Diphenylderivat ein Diphenylderivat gemäß den nachstehenden Formeln (III-A) sowie dessen Salze ist: 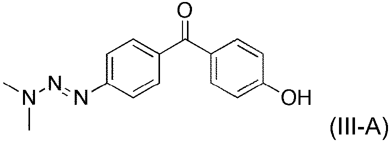 oder
wobei das Diphenylderivat ein Diphenylderivat gemäß der nachstehenden Formel (VI-A) sowie dessen Salze ist: 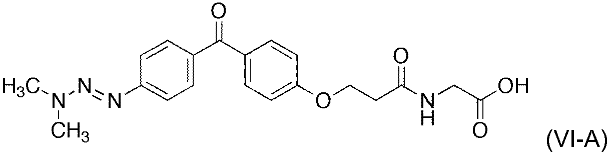 oder
wobei das Diphenylderivat ein Diphenylderivat gemäß der nachstehenden Formel (VIII-A) sowie dessen Salze ist:

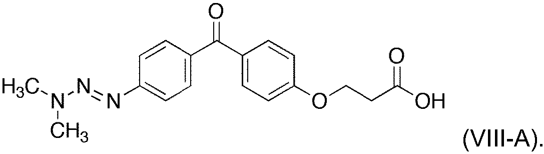

Die erfindungsgemäß eingesetzten Wirkstoffe bzw. Verbindungen (d. h. Diphenylderivate) als solche sowie deren Herstellung bzw. Bereitstellung sind grundsätzlich aus dem Stand der Technik bekannt (vgl. insbesondere die Dokumente WO 2012/122969 A1, DE 10 2015 008 631 A1, EP 2 557 075 A1 und WO 2009/004060 A1).

Die dort beschriebenen Wirkstoffe bzw. Verbindungen (d. h. Diphenylderivate der vorgenannten Art) sind bislang aber ausschließlich für die Krebs- und Tumortherapie in Betracht gezogen bzw. angewendet worden.

Wie eingangs bereits beschreiben, hat nunmehr erstmals - vollkommen überraschend und unerwartet -die Anmelderin gefunden, dass diese speziellen Wirkstoffe auf Basis von Diphenylderivaten sich auch in effizienter Weise für die bevorzugt kausale Therapie von Viruserkrankungen bzw. viralen Infektionen (Virusinfektionen), insbesondere Infektionen mit Corona-Viren, insbesondere COVID-19, eignen.

Viele Viren nutzen und manipulieren bestimmte Signal- bzw. Kommunikationswege ihrer Wirtszellen, um ihre eigene Vermehrung zu befördern. Die Anmelderin hat nunmehr in vollkommen überraschender wie unerwarteter Weise herausgefunden, dass mittels der in Rede stehenden Diphenylderivate die betreffenden viralen Signal- bzw. Kommunikationswege gestört bzw. unterbrochen werden können und eine solche Unterbrechung dieser Signalwege dazu führt, dass sich die Viren nicht mehr vermehren können.

Ein Vorteil der Tatsache, dass die Anmelderin dabei auf an sich bekannte, aber für den erfindungsgemäßen Einsatz- bzw. Therapiezweck überhaupt noch nicht in Betracht gezogene Wirkstoffe zurückgreifen kann, dass diese Wirkstoffe ohne Weiteres zugänglich sind und vor allem einen entscheidenden Entwicklungsvorsprung vor neuartigen Wirkstoffen haben, da bereits zahlreiche Eigenschaften der Wirkstoffe untersucht worden sind, insbesondere Stabilitätsverhalten, Toxizitäten, pharmazeutisch-pharmakologische Formulierbarkeiten und Applikationsarten etc. Daher können etwaige klinische Studien zu Zwecken der erfindungsgemäßen antiviralen Therapie grundsätzlich unmittelbar und ohne weitergehende umfangreiche Voruntersuchungen einsetzen.

Wie die Anmelderin herausgefunden hat, führen die erfindungsgemäß eingesetzten Diphenylderivate insbesondere dazu (ohne sich jedoch auf eine bestimmte Theorie festlegen zu wollen), dass in den Zellen bestimmte Reparatursysteme runterreguliert werden. Diese Systeme werden auch von Viren genutzt, um einen sogenannten DNA-Damage-Response (DDR) zu erzeugen, welcher die Wirtszelle in Zellzyklusarrest und Apoptose treibt, wobei die dabei freiwerdenden Zerfallsprodukte von den Viren genutzt werden, um ihre eigene Vermehrung zu betreiben. Durch die Runterregulierung dieser Zellreparatursysteme wird den Viren die Möglichkeit zur Vermehrung genommen, so dass auf diese Basis unter Verwendung der erfindungsgemäß eingesetzten Diphenylderivate erstmals eine effiziente, insbesondere kausale, Therapie von Viruserkrankungen bzw. viralen Infektionen (Virusinfektionen), insbesondere Infektionen mit Corona-Viren, insbesondere COVID-19, bereitgestellt werden kann.

Mit anderen Worten hat die Anmelderin überraschend gefunden, dass der zuvor beschriebene Shutdown der Reparatur(mechanismen) der Zelle nicht nur Tumorzellen schädigt, sondern auch die Virusreplikation in infizierten Wirtszellen blockiert, wenn man den Viren die Möglichkeit nimmt, auf diese Systeme zuzugreifen.

Konkrete Untersuchungen bezüglich des Shutdown der Reparatur(mechanismen) der Zelle wurden seitens der Anmelderin insbesondere an Ovarialkarzinom-Krebszellen bzw. Ovarialkarzinom-Krebszell-Linien sowie aber auch an weiteren Zellsystemen und betreffenden Modellen durchgeführt.

Aufgrund des zuvor aufgezeigten Wirkmechanismus stellen die erfindungsgemäß eingesetzten Diphenylderivate erstmals eine effiziente, insbesondere kausale, antivirale Breitband-Therapie bereit bzw. eignen sich die erfindungsgemäß eingesetzten Diphenylderivate unerwartet als antivirales Breitbandmittel (d. h. in Bezug auf die Therapie von Infektionen durch eine Vielzahl von Viren, insbesondere aber auch Infektionen mit Corona-Viren, insbesondere COVID-19, da insbesondere letztgenannte auf die zuvor beschriebenen Reparatur(mechanismen) der Wirtszellen zurückgreifen).

Die erfindungsgemäß eingesetzten Diphenylderivate zeichnen sich gegenüber anderen antiviralen Wirkstoffen (z. B. Remdesivir etc.) und insbesondere auch gegenüber anderen Diphenylderivaten durch eine bessere therapeutische Breite, insbesondere aber auch durch geringere Nebenwirkungen bei hoher antiviraler Wirkung, insbesondere auch durch eine verbesserte Aufnahme in Zellen, welche bevorzugt von Coronaviren bzw. Viren des SARS-Typs befallen werden (z. B. Lunge, Nieren, Herz, CNS etc.), aus.

Die erfindungsgemäß eingesetzten Diphenylderivate sind insbesondere mit einer Triazengruppe ausgerüstet. Derartige Triazenderivate sind aber bislang nur als Wirkstoffe mit antitumoraler Wirksamkeit bekannt geworden (z. B. Dacarbazin oder Temozolomid), sind aber selbst als Zytostatikum niemals zu einer breiteren Anwendung gekommen (d. h. Melanom bzw. Glioblastom); eine Indikation außerhalb der antitumoralen Wirksamkeit ist bislang unerkannt geblieben und daher nicht in Betracht gezogen worden.

Die erfindungsgemäß eingesetzten Diphenylderivate besitzen - neben ihrer antitumoralen Wirkung - vollkommen überraschend auch eine ausgeprägte breite antivirale Wirkung (z. B. DNA-, RNA-Viren), insbesondere in Bezug auf RNA-Viren (z. B. SARS-basierte Viren). Diese Verbindungen zeichnen sich insbesondere dadurch aus, dass sie eine sehr gute Verträglichkeit aufweisen und für die Therapie beim Menschen und am Tier zugänglich gemacht werden können, insbesondere zur Therapie von schweren Viruserkrankungen des SARS-Typs in der akuten Phase bzw. zur Verhinderung oder Eindämmung einer Infektion und der Ausbreitung der Viren im Körper, zur Vermeidung lebensbedrohlicher Komplikationen mit Langzeitschädigungen und Todesfolge etc.

Ohne sich auf eine bestimmte Theorie festlegen zu wollen, beruht die neuartige medizinische Indikation bzw. Wirkungsweise der im Rahmen der vorliegenden Erfindung erstmals als antiviral wirksame Wirkstoffe eingesetzten Diphenylderivate insbesondere darauf, dass die erfindungsgemäß eingesetzten Verbindungen in therapeutisch wirksamen Dosen bzw. Konzentrationen (z. B. 10-1.000 µmol/l) die DNA-Expression von Genen im Nucleus der Wirtszelle unterdrücken, welche für eine Reihe von wichtigen Reparatursystemen (HR; NER;...) kodieren, sodass es dem eindringenden Virus, insbesondere RNA-Virus, nicht gelingt, auf DNA-Ebene im Nucleus der Wirtszelle den entscheidenden metabolischen Shift zu erzeugen, nämlich einen Reparatursystem vermittelten DNA-Damage-Response (DDR) auszulösen und infolgedessen einen Zellzyklus-Arrest und eine Apoptose zu erzwingen, der bzw. die im Allgemeinen zum Absterben der Wirtszelle führt. Durch die Blockade bzw. Nichtexpression wichtiger Reparaturgene durch die erfindungsgemäß eingesetzten Verbindungen wird dem Virus somit die Grundlage entzogen, diese für seine eigene Vermehrung essentiellen Prozesse zu nutzen.

Durch die Behandlung der Wirtszellen mit den erfindungsmäßig eingesetzten Verbindungen kommt es somit zu einem Shutdown wichtiger Reparatursysteme, welche das Virus aber benötigt, um seine eigene Vermehrung in Gang zu bringen und aufrechtzuerhalten. Bereits die Blockade eines einzigen spezifischen Reparatursystems hat signifikant hemmende Auswirkungen auf die Virusvermehrung. Überraschend ist vor allem, dass die erfindungsgemäß eingesetzten Verbindungen temporär breit in das Expressionsgeschehen einer Reihe von wichtigen Reparatursystemen eingreifen und somit die befallene Wirtszelle vor dem durch das Virus induzierten Transformationsprozess der Wirtszelle geschützt wird.

Die vorliegende Erfindung und das erfindungsgemäße Therapiekonzept sind folglich mit einer Vielzahl von Vorteilen und Besonderheiten verbunden.

Was das erfindungsgemäß zur Verwendung bei der prophylaktischen und/oder therapeutischen Behandlung von Viruserkrankungen, insbesondere Corona-Infektionen, insbesondere COVID-19, eingesetzte Diphenylderivat anbelangt betrifft, so enthält - wie zuvor beschrieben - das Diphenylderivat pro Molekül (i) mindestens eine Dialkyltriazenylgruppe sowie (ii) mindestens eine Sulfooxygruppe (vgl. auch Patentanspruch 1).

Wie zuvor dargelegt, ist Gegenstand der vorliegenden Erfindung - gemäß einem ersten Aspekt der vorliegenden Erfindung - somit ein Diphenylderivat zur Verwendung bei der prophylaktischen und/oder therapeutischen Behandlung von Viruserkrankungen, insbesondere Corona-Infektionen, insbesondere COVID-19,
wobei das Diphenylderivat ein Diphenylderivat gemäß der nachstehenden Formel (IV-A1) oder (IV-A2) ist: 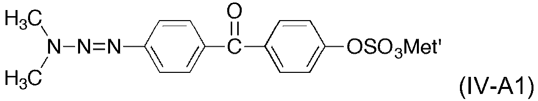 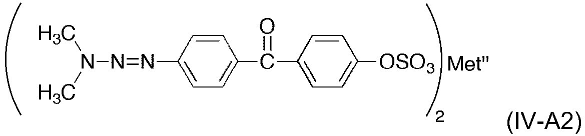
worin:
   Met' ein einwertiges Metallion, insbesondere ein Alkalimetallion, vorzugsweise ein Natriumion, bezeichnet und
   Met" ein zweiwertiges Metallion, insbesondere ein Erdalkalimetallion, bezeichnet; oder
   wobei das Diphenylderivat ein Diphenylderivat gemäß der nachstehenden Formel (IV-A3) ist: 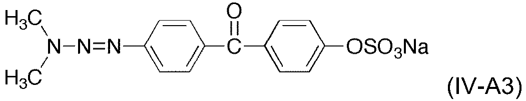 oder
   wobei das Diphenylderivat ein Diphenylderivat gemäß der nachstehenden Formel (IV-A) sowie dessen Salze ist: 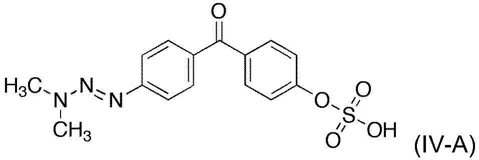 oder
   wobei das Diphenylderivat ein Diphenylderivat gemäß den nachstehenden Formeln (IV-B) sowie dessen Salze ist: 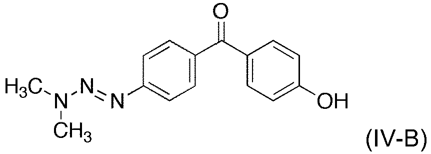 oder
   wobei das Diphenylderivat ein Diphenylderivat gemäß den nachstehenden Formeln (III-A) sowie dessen Salze ist: 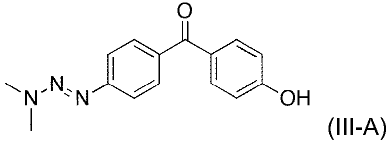 oder
   wobei das Diphenylderivat ein Diphenylderivat gemäß der nachstehenden Formel (VI-A) sowie dessen Salze ist: 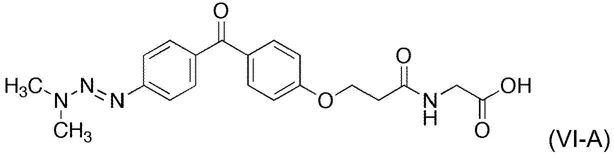 oder
   wobei das Diphenylderivat ein Diphenylderivat gemäß der nachstehenden Formel (VIII-A) sowie dessen Salze ist: 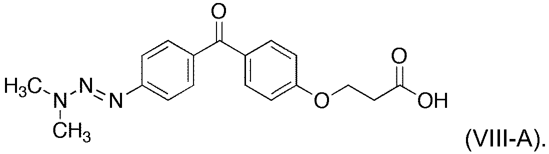
   Gemäß dem vorliegenden ersten Erfindungsaspekt ist Gegenstand der vorliegenden Erfindung insbesondere gleichermaßen ein Diphenylderivat, insbesondere Benzophenonderivat, zur Verwendung bei der prophylaktischen und/oder therapeutischen Behandlung von Viruserkrankungen, insbesondere Corona-Infektionen, insbesondere COVID-19, insbesondere ein Diphenylderivat wie zuvor definiert,
   wobei das Diphenylderivat, insbesondere Benzophenonderivat, ein Diphenylderivat gemäß den nachstehenden Formeln (III-A) ist: 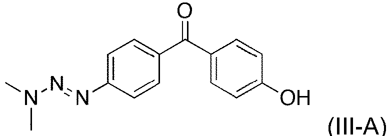 sowie dessen Salze.

Gemäß dem vorliegenden ersten Erfindungsaspekt ist somit auch Gegenstand der vorliegenden Erfindung insbesondere gleichermaßen ein Diphenylderivat, insbesondere Benzophenonderivat, zur Verwendung bei der prophylaktischen und/oder therapeutischen Behandlung von Viruserkrankungen, insbesondere Corona-Infektionen, insbesondere COVID-19, insbesondere ein Diphenylderivat wie zuvor definiert, wobei das Diphenylderivat, insbesondere Benzophenonderivat, ein Diphenylderivat gemäß den nachstehenden Formeln (IV-A) bis (IV-B) ist: 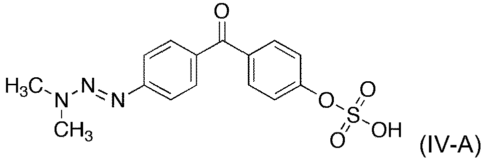 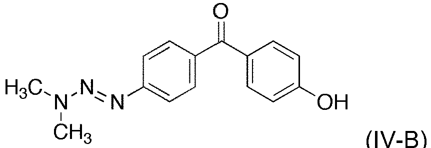 sowie dessen Salze.

Gemäß dem vorliegenden ersten Erfindungsaspekt ist Gegenstand der vorliegenden Erfindung somit insbesondere ein Diphenylderivat, insbesondere Benzophenonderivat, zur Verwendung bei der prophylaktischen und/oder therapeutischen Behandlung von Viruserkrankungen, insbesondere Corona-Infektionen, insbesondere COVID-19, insbesondere ein Diphenylderivat wie zuvor definiert,
wobei das Diphenylderivat, insbesondere Benzophenonderivat, ein Diphenylderivat gemäß der nachstehenden Formel (IV-A1) oder (IV-A2) ist: 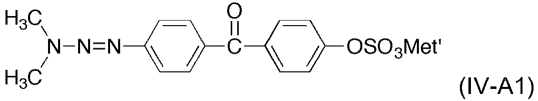 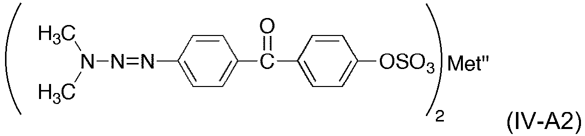
worin:
   Met' ein einwertiges Metallion, insbesondere ein Alkalimetallion, vorzugsweise ein Natriumion, bezeichnet und
   Met" ein zweiwertiges Metallion, insbesondere ein Erdalkalimetallion, bezeichnet.

Gemäß dem vorliegenden ersten Erfindungsaspekt ist somit auch Gegenstand der vorliegenden Erfindung insbesondere gleichermaßen ein Diphenylderivat, insbesondere Benzophenonderivat, zur Verwendung bei der prophylaktischen und/oder therapeutischen Behandlung von Viruserkrankungen, insbesondere Corona-Infektionen, insbesondere COVID-19, insbesondere ein Diphenylderivat wie zuvor definiert, wobei das Diphenylderivat, insbesondere Benzophenonderivat, ein Diphenylderivat gemäß der nachstehenden Formel (IV-A3) ist:

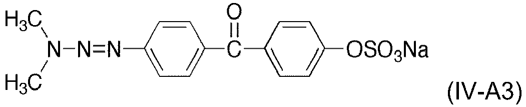

Gemäß dem vorliegenden ersten Erfindungsaspekt ist somit auch Gegenstand der vorliegenden Erfindung insbesondere gleichermaßen ein Diphenylderivat, insbesondere Benzophenonderivat, zur Verwendung bei der prophylaktischen und/oder therapeutischen Behandlung von Viruserkrankungen, insbesondere Corona-Infektionen, insbesondere COVID-19, insbesondere ein Diphenylderivat wie zuvor definiert, wobei das Diphenylderivat, insbesondere Benzophenonderivat, ein Diphenylderivat gemäß der nachstehenden Formel (IV-A) ist:

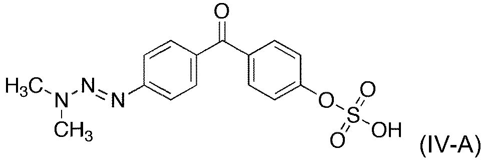

Gemäß dem vorliegenden ersten Erfindungsaspekt ist somit auch Gegenstand der vorliegenden Erfindung insbesondere gleichermaßen ein Diphenylderivat, insbesondere Benzophenonderivat, zur Verwendung bei der prophylaktischen und/oder therapeutischen Behandlung von Viruserkrankungen, insbesondere Corona-Infektionen, insbesondere COVID-19, insbesondere ein Diphenylderivat wie zuvor definiert, wobei das Diphenylderivat, insbesondere Benzophenonderivat, ein Diphenylderivat gemäß der nachstehenden Formel (VI-A) ist: 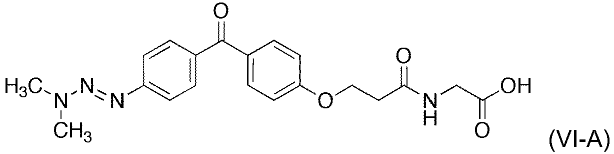 sowie dessen Salze.

Schließlich - gemäß dem vorliegenden ersten Erfindungsaspekt - ist auch Gegenstand der vorliegenden Erfindung insbesondere gleichermaßen ein Diphenylderivat, insbesondere Benzophenonderivat, zur Verwendung bei der prophylaktischen und/oder therapeutischen Behandlung von Viruserkrankungen, insbesondere Corona-Infektionen, insbesondere COVID-19, insbesondere ein Diphenylderivat wie zuvor definiert, wobei das Diphenylderivat, insbesondere Benzophenonderivat, ein Diphenylderivat gemäß der nachstehenden Formel (VIII-A) ist: 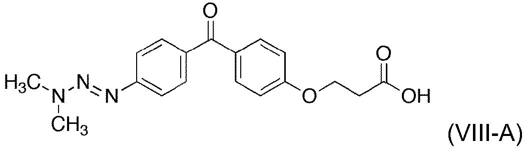 sowie dessen Salze.

Bezüglich des ersten Erfindungsaspekts, insbesondere im Hinblick auf sämtliche zuvor beschriebene Ausführungsformen, ist insbesondere weiterhin auszuführen, dass bevorzugt das Diphenylderivat systemisch appliziert und angewendet wird.

Bezüglich des ersten Erfindungsaspekts, insbesondere im Hinblick auf sämtliche zuvor beschriebene Ausführungsformen, ist insbesondere weiterhin auch noch auszuführen, dass optional das Diphenylderivat, insbesondere das das Diphenylderivat enthaltende Arzneimittel oder Medikament, zusammen mit mindestens einem weiteren Wirkstoff angewendet oder appliziert werden kann, wobei der mindestens eine weitere Wirkstoff insbesondere ausgewählt sein kann aus entzündungshemmenden Wirkstoffen (insbesondere Kortikosteroiden, wie z. B. Dexamethason), blutverdünnenden Wirkstoffen, antiviral wirkenden Wirkstoffen sowie deren Kombinationen.

Vorliegend beschrieben ist auch die Verwendung eines Diphenylderivats, wie zuvor definiert, zur prophylaktischen und/oder therapeutischen Behandlung von Viruserkrankungen, insbesondere Corona-Infektionen, insbesondere COVID-19, bzw. die erfindungsgemäße Verwendung der vorgenannten Wirkstoffe bzw. Diphenylderivate zur Herstellung eines Arzneimittels oder Medikaments zur prophylaktischen und/oder therapeutischen Behandlung von viralen Infektionen (Virusinfektionen), insbesondere Infektionen mit Corona-Viren, insbesondere COVID-19.

Im Rahmen der vorliegend beschriebenen Verwendung ist es insbesondere vorgesehen, dass der Wirkstoff bzw. das Diphenylderivat systemisch appliziert wird.

Weiterhin kann es im Rahmen der vorliegend beschriebenen Verwendung erforderlichenfalls vorgesehen sein, dass der Wirkstoff bzw. das Diphenylderivat zusammen mit mindestens einem weiteren Wirkstoff angewendet oder appliziert wird. Der weitere Wirkstoff kann insbesondere ausgewählt sein aus entzündungshemmenden Wirkstoffen (insbesondere Kortikosteroiden, wie z. B. Dexamethason), blutverdünnenden Wirkstoffen, antiviral wirkenden Wirkstoffen sowie deren Kombinationen. Der weitere Wirkstoff wird insbesondere gleichermaßen systemisch angewendet bzw. appliziert.

Für weitergehende Einzelheiten zu der vorliegend beschriebenen Verwendung kann Bezug genommen werden auf die vorstehenden Ausführungen zum ersten Erfindungsaspekt, wobei diese Ausführungen gleichermaßen für die vorliegend beschriebene Verwendung gelten.

Vorliegend beschrieben ist zudem die Verwendung eines Diphenylderivats, wie zuvor definiert, als Antivirusmittel zur prophylaktischen und/oder therapeutischen Behandlung von Viruserkrankungen, insbesondere Corona-Infektionen, insbesondere COVID-19, bzw. zur Herstellung eines Antivirusmittel zur prophylaktischen und/oder therapeutischen Behandlung von Viruserkrankungen, insbesondere Corona-Infektionen, insbesondere COVID-19.

Im Rahmen der vorliegend beschriebenen Verwendung ist es insbesondere vorgesehen, dass der Wirkstoff bzw. das Diphenylderivat systemisch appliziert wird.

Weiterhin kann es im Rahmen der vorliegend beschriebenen Verwendung erforderlichenfalls vorgesehen sein, dass der Wirkstoff bzw. das Diphenylderivat zusammen mit mindestens einem weiteren Wirkstoff angewendet oder appliziert wird. Der weitere Wirkstoff kann insbesondere ausgewählt sein aus entzündungshemmenden Wirkstoffen (insbesondere Kortikosteroiden, wie z. B. Dexamethason), blutverdünnenden Wirkstoffen, antiviral wirkenden Wirkstoffen sowie deren Kombinationen. Der weitere Wirkstoff wird insbesondere gleichermaßen systemisch angewendet bzw. appliziert.

Für weitergehende Einzelheiten zu der vorliegend beschriebenen Verwendung kann Bezug genommen werden auf die vorstehenden Ausführungen zu den zuvor beschriebenen Aspekten, wobei diese Ausführungen gleichermaßen für die vorliegend beschriebene Verwendung gelten.

Vorliegend beschrieben ist weiterhin ein Verfahren zur prophylaktischen und/oder therapeutischen Behandlung von Viruserkrankungen, insbesondere Corona-Infektionen, insbesondere COVID-19, wobei bei dem Verfahren einem Patienten eine pharmazeutisch wirksame bzw. therapeutisch wirksame Menge eines Diphenylderivats, wie zuvor definiert, verabreicht wird.

Im Rahmen desvorliegend beschriebenen Verfahrens gemäß diesem Erfindungsaspekt ist es insbesondere vorgesehen, dass der Wirkstoff bzw. das Diphenylderivat systemisch appliziert wird.

Weiterhin kann es im Rahmen des vorliegend beschriebenen Verfahrens erforderlichenfalls vorgesehen sein, dass der Wirkstoff bzw. das Diphenylderivat zusammen mit mindestens einem weiteren Wirkstoff angewendet oder appliziert wird. Der weitere Wirkstoff kann insbesondere ausgewählt sein aus entzündungshemmenden Wirkstoffen (insbesondere Kortikosteroiden, wie z. B. Dexamethason), blutverdünnenden Wirkstoffen, antiviral wirkenden Wirkstoffen sowie deren Kombinationen. Der weitere Wirkstoff wird insbesondere gleichermaßen systemisch angewendet bzw. appliziert.

Für weitergehende Einzelheiten zudem vorliegend beschriebenen Verfahren kann Bezug genommen werden auf die vorstehenden Ausführungen zu den zuvor beschriebenen Aspekten, wobei diese Ausführungen gleichermaßen für das vorliegend beschriebene Verfahren gelten.

Ebenfalls Gegenstand der vorliegenden Erfindung - gemäß einem **zweiten** Aspekt der vorliegenden Erfindung - ist ein Arzneimittel oder Medikament zur Verwendung bei der prophylaktischen und/oder therapeutischen Behandlung von Viruserkrankungen, insbesondere Corona-Infektionen, insbesondere COVID-19, insbesondere Antivirusmittel, wobei das Arzneimittel oder Medikament, insbesondere Antivirusmittel, mindestens ein Diphenylderivat, wie zuvor definiert, zusammen mit einem pharmazeutisch verträglichen Träger oder Exzipienten enthält.

Auch im Rahmen dieses Erfindungsaspekt ist es insbesondere vorgesehen, dass der Wirkstoff bzw. das Diphenylderivat systemisch appliziert wird.

Weiterhin kann es im Rahmen der erfindungsgemäßen Verwendung gemäß diesem Erfindungsaspekt erforderlichenfalls vorgesehen sein, dass der Wirkstoff bzw. das Diphenylderivat zusammen mit mindestens einem weiteren Wirkstoff angewendet oder appliziert wird. Der weitere Wirkstoff kann insbesondere ausgewählt sein aus entzündungshemmenden Wirkstoffen (insbesondere Kortikosteroiden, wie z. B. Dexamethason), blutverdünnenden Wirkstoffen, antiviral wirkenden Wirkstoffen sowie deren Kombinationen. Der weitere Wirkstoff wird insbesondere gleichermaßen systemisch angewendet bzw. appliziert.

Für weitergehende Einzelheiten zu diesem Erfindungsaspekt kann Bezug genommen werden auf die vorstehenden Ausführungen zu den zuvor beschriebenen Erfindungsaspekten, wobei diese Ausführungen gleichermaßen für den vorliegenden Erfindungsaspekt gelten.

Schließlich ist Gegenstand der vorliegenden Erfindung - gemäß einem **dritten** Aspekt der vorliegenden Erfindung - gleichermaßen eine pharmazeutische Kombination zur (Verwendung bei der) prophylaktischen und/oder therapeutischen Behandlung von Viruserkrankungen, insbesondere Corona-Infektionen, insbesondere COVID-19,
wobei die pharmazeutische Kombination
   (A) mindestens ein Diphenylderivat, wie zuvor definiert bzw. wie in einem der Ansprüche 1, 3 und 6 definiert; sowie
   (B) mindestens einen weiteren Wirkstoff, ausgewählt aus entzündungshemmenden Wirkstoffen, insbesondere Kortikosteroiden, blutverdünnenden Wirkstoffen, antiviral wirkenden Wirkstoffen sowie deren Kombinationen,
umfasst.

Auch im Rahmen dieses Erfindungsaspekt ist es insbesondere vorgesehen, dass der Wirkstoff bzw. das Diphenylderivat systemisch appliziert wird.

Auch der weitere Wirkstoff wird insbesondere gleichermaßen systemisch angewendet bzw. appliziert.

Für weitergehende Einzelheiten zu diesem Erfindungsaspekt kann Bezug genommen werden auf die vorstehenden Ausführungen zu den zuvor beschriebenen Erfindungsaspekten, wobei diese Ausführungen gleichermaßen für den vorliegenden Erfindungsaspekt gelten.

Die erfindungsgemäß eingesetzten Wirkstoffe bzw. die erfindungsgemäß eingesetzten Diphenylderivate (wie zuvor definiert) besitzen zudem insbesondere ein allgemeines (d. h. allgemeingültiges) bzw. umfassendes bzw. virusunspezifisches antivirales Wirkpotential und/oder Wirkspektrum. Folglich können die erfindungsgemäß eingesetzten Wirkstoffe bzw. die erfindungsgemäß eingesetzten Diphenylderivate dazu verwendet werden, nahezu alle Arten von Viruserkrankungen zu therapieren. Dies gilt für alle zuvor beschriebenen Erfindungsaspekte (d. h. erster bis sechster Aspekt der vorliegenden Erfindung, wie zuvor beschrieben). So können - gemäß allen zuvor beschriebenen Erfindungsaspekten - die erfindungsgemäß eingesetzten Wirkstoffe bzw. die erfindungsgemäß eingesetzten Diphenylderivate (wie zuvor definiert) für die prophylaktische und/oder therapeutische Behandlung von Viruserkrankungen eingesetzt bzw. verwendet werden, wobei die Viruserkrankungen insbesondere ausgewählt sein können aus der Gruppe von Corona-Infektionen, wie MERS und SARS, insbesondere COVID-19, HIV-Infektionen, Influenza, Parainfluenza, Cytomegalievirus-Infektionen (HCMV-Infektionen), Papillomavirus-Infektionen (HPV-Infektionen), viralen Hepatitiden (Virus-Hepatitiden), Enterovirus-Infektionen, Epstein-Barr-Virus-Infektionen, Herpesviren-Infektionen, Varizellavirus-Infektionen, insbesondere Varizella-zoster-Virus-Infektionen, Adenovirus-Infektionen, Rotavirus-Infektionen, Gelbfiebervirus-Infektionen, viralem hämorrhagischem Fieber (VHF), FSME-Virus-Infektionen, West-Nil-Virus-Infektionen, Ebolavirus-Infektionen, Tollwut (Rabies), Marburgvirus-Infektionen, Hantavirus-Infektionen, Lassa-Virus-Infektionen, Dengue-Virus-Infektionen, Arena-Virus-Infektionen, Arbovirus-Infektionen, Coxsackie-Virus-Infektionen, Masern-, Mumps, Röteln- und Ringelröteln-Virus-infektionen sowie Poliovirus-Infektionen, vorzugsweise aus der Gruppe von Corona-Infektionen, insbesondere COVID-19, HIV-Infektionen, Influenza, Parainfluenza, Cytomegalievirus-Infektionen (HCMV-Infektionen), Papillomavirus-Infektionen (HPV-Infektionen) und viralen Hepatitiden (Virus-Hepatitiden), besonders bevorzugt aus der Gruppe von Corona-Infektionen, insbesondere COVID-19.

Weitere Ausgestaltungen, Abwandlungen und Variationen - soweit sie von den Ansprüchen gedeckt sind - sowie Vorteile der vorliegenden Erfindung sind für den Fachmann beim Lesen der Beschreibung ohne Weiteres erkennbar und realisierbar, ohne dass er dabei den Rahmen der vorliegenden Erfindung verlässt.

Die folgenden Ausführungsbeispiele dienen lediglich der Veranschaulichung der vorliegenden Erfindung, jedoch ohne die vorliegende Erfindung hierauf zu beschränken.

### Ausführungsbeispiele

### 1. Shutdown zelleigener Reparatursysteme als neuer Ansatz für antivirale Therapeutika

Im Rahmen der vorliegenden Erfindung ist überraschend herausgefunden worden, dass die erfindungsgemäß eingesetzten Diphenylderivate eine antivirale Wirkung besitzen. Ohne sich hierbei auf diese Theorie beschränken zu wollen, wird davon ausgegangen, dass die antivirale Wirkweise auf einer Herunterregulation bestimmter zellulärer Reparatursysteme basiert.

In diesem Zusammenhang wurden erste Versuche mit einem Diphenylderivat gemäß der obigen Formel (IV-A3) (wobei dieses Diphenylderivat, welches gleichermaßen ein Triazen ist, nachfolgend einfach nur als "Verbindung (IV-A3)" bezeichnet wird) sowie mit einem Diphenylderivat gemäß der obigen Formel (III-A) (wobei dieses Diphenylderivat, welches ebenfalls auch ein Triazen darstellt, nachfolgend einfach nur als "Verbindung (III-A)" bezeichnet wird) 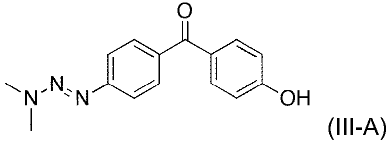 durchgeführt.

Darüber hinaus wurden weitere Versuche mit den Diphenylderivaten gemäß den Strukturen der ursprünglichen Patentansprüche 13, 14, 16, 18 und 19 durchgeführt, welche im Wesentlichen gleiche Ergebnisse liefern.

Der Einfachheit halber sind nachfolgend aber lediglich die Versuchsergebnisse zu Verbindung (IV-A3) und Verbindung (III-A) referiert.

Verbindung (IV-A3) induzierte sehr stark die CDKN1A-Transkription (Cyclinabhängige-Kinase Inhibitor 1A), was auf eine Blockade des Zellzyklus als erste molekulare Wirkungsweise hindeutet. Darüber hinaus vermindert die Substanz (Verbindung (IV-A3)) die Expression bestimmter Gen-Abschnitte, welche an der Regulation bestimmter Reparaturmechanismen der DNA beteiligt sind, wie z. B. Homologe Rekombination (HR), Dealkylierung der DNA, Mismatch-Reparatur (MMR) bzw. Nukleotid-Exzisions-Reparatur (NER).

RNA-Isolierung und Gesamt Transkriptom Sequenzierung:
Für die Gesamt-Transkriptom-Sequenzierung (RNA-Seq) wurden unterschiedliche Ansätze von Zellen (3,5 x 10-6 Zellen UWB1.289 oder 3,3 x 10-6 Zellen UWB1.289 + BRCA1) in T75-Kulturgefäßen angezüchtet. Nach 24 Stunden wurde das Medium entfernt und durch neues Medium mit 1.000 µM der Verbindung (IV-A3) bzw. 40 µM der Verbindung (III-A) ersetzt. Standard-Kulturmedium diente als Kontrolle. Nach 24 Stunden Substanzeinwirkung wurden die Zellen geerntet und die RNA nach dem üblichen miRNeasy-Verfahren (Qiagen, Hilden, Deutschland) extrahiert. Um Kontaminationen mit restlicher genomischer DNA zu entfernen, wurde ein Säulen-Reinigungsschritt durchgeführt. Alle Experimente wurden dreifach durchgeführt und wurden nach den bestehenden Vorschriften der Hersteller ausgewertet. Für die Erstellung einer Gen-Bibliothek wurde der TruSeq Stranded mRNA Lbrary Prep Kit (Illumina) nach der Vorschrift des Herstellers verwendet, beginnend mit 1 µg Gesamt-RNA. Die kodierten Bibliotheken (barcoded) wurden gepoolt und auf einer Illumina NextSeq500 Plattform sequenziert.

Bioinformatische Analyse der Transkriptom-Ergebnisse:
Die Auswertung erfolgte unter Verwendung der Software Trimmomatic und STAR. Hierarchische Cluster wurden unter Verwendung der sogenannten Euklidabstand- und Assoziationsbildung durchgeführt und mit Complex Heatmap visualisiert. Weiterhin wurde eine Principal-Komponenten-Analyse (Hauptkomponentenanalyse bzw. PCA-Plot) unter Verwendung des R Stats package der Firma R-Institut für statistisches Rechnen, Wien, Österreich, durchgeführt. Die Methode DESeq2 wurde angewendet, um differentiell exprimierte Gene nach Standardkriterien zu identifizieren. Es wurden nur Gene mit multipel korrigierten p-Werten von kleiner 0.05 (padj von DESeq2) als signifikant angesehen.

Transkriptionsprofile von mit Verbindung (IV-A3) und Verbindung (III-A) behandelten Ovarialkarzinomzellen:
Um den Wirkmechanismus der erfindungsgemäß eingesetzten Diphenylderivate bzw. Triazene bei Ovarial-Krebs zu untersuchen, wurde eine umfassende Genexpressionsanalyse bei UWB1.289 Zellpaaren, die mit Verbindung (IV-A3) bzw. Verbindung (III-A) behandelt worden waren, erstellt. Es wurden im doppelten Ansatz Proben von BRCA1-defizienten und BRAC1-profizienten UWB1.289 Zellen einer Kurzzeitbehandlung unterzogen (1.000 µM Verbindung (IV-A3) bzw. 40 µM Verbindung (III-A) über 24 Stunden). Zum Zeitpunkt der RNA-Isolierung und der Transkriptom-Sequenzierung wurden keine Anzeichen für morphologische Veränderungen an den Zellen gefunden. Es zeigte sich, dass sich bei der hierarchischen Gruppierung und der Hauptkomponenten-Analyse bei beiden Zelllinien ein deutlicher Unterschied in den Transkriptom Profilen zwischen den beiden Zelllinien und abhängig von den eingesetzten Wirkstoffen (Verbindung (IV-A3) bzw. Verbindung (III-A)) ergab. Darüber hinaus zeigten mit Verbindung (IV-A3) behandelte Zellen größere Unterschiede zu den nicht behandelten Kontrollen im PCA-Plot im Vergleich zu den mit Verbindung (III-A) behandelten Zellen. Dies weist darauf hin, dass Verbindung (IV-A3) stärker ausgeprägte Veränderungen im Transkriptom induziert als Verbindung (III-A). Nichtsdestotrotz bewirken ungeachtet der Unterschiede beide Verbindungen eine ausreichende Veränderung des Transkriptoms, um von einem therapeutischen Effekt ausgehen zu können. Dieser Befund wurde weiter dadurch unterstützt, dass bei Verbindung (IV-A3) eine höhere Zahl differentiell transkribierter Gene gefunden wurden im Vergleich zu Verbindung (III-A) und den Kontrollzellen.

Darüber hinaus wurde eine differentielle Genexpressionsanalyse durchgeführt mit Schwerpunkt auf solche Gene, die an der Regulation des Zellzyklus, des Drug-Membrantransports, des Metabolismus, der zellulären Adhäsion und anderer krankheitsbedingter Stoffwechselwege beteiligt sind, wie z. B. Fibroblasten-Wachstumsfaktor, kurz FGF bzw. dem SMAD-Signalweg). Es wurden die Schnittmengen der in beiden Zelllinien differentiell exprimierten Genen nach Substanzbehandlung ermittelt (log2FC-Werte).

Eine Behandlung mit Verbindung (IV-A3) führt zu einer deutlichen Herunterregulierung verschiedener Gene aus der CDK-Familie und gleichzeitig zu einer starken Induktion von CDKN1A (log2FC = 4.49).

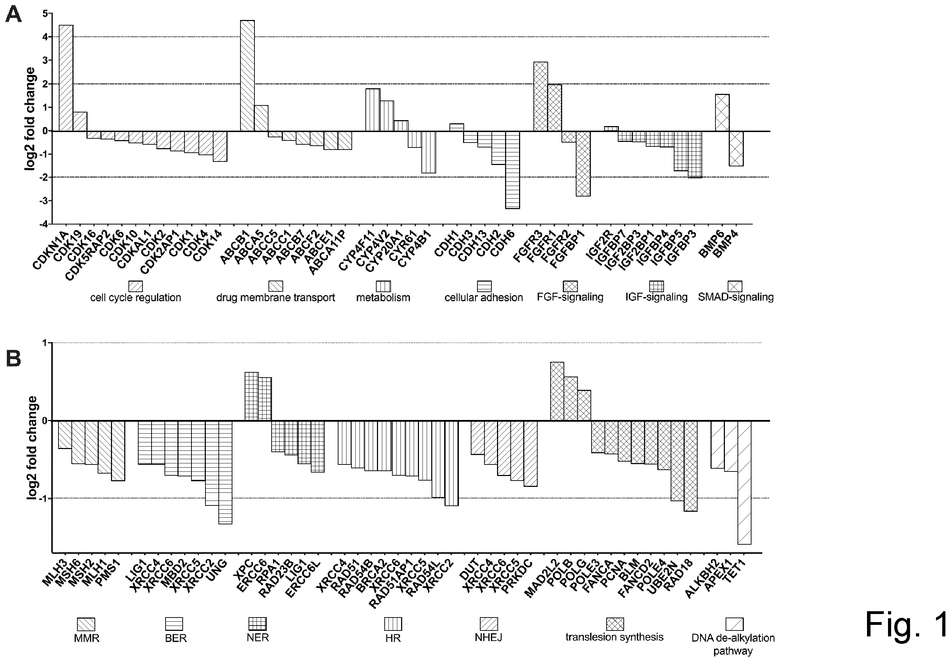

Dies deutet auf eine Aktivierung des Cyclin-abhängigen Proteinkinase-Inhibitor-Stoffwechselwegs und eine Arretierung des Zellzyklus als frühe Reaktion auf die Behandlung hin. In Übereinstimmung mit der nach Behandlung mit Verbindung (IV-A3) verstärkten Zellablösung wurden einige Gene der Cadherin-Zell-Adhäsions-Moleküle, insbesondere CDH6, runterreguliert (vgl. Figur 1A).

Neben bestimmten genregulatorischen Effekten von Verbindung (IV-A3) und Verbindung (III-A) auf die Expression von Drug-Efflux-Pumpenmechanismen (ABC-Transporter-Familie) und auf einige Cytochrom-Enzyme wurde festgestellt, dass insbesondere eine Reihe von DNA-Reparatursystemen, z. B. die Mismatch-Reparatur (MMR), die Basen-Exzisions-Reparatur (BER; XRCC2), die Nukleotid-Exzisions-Reparatur (NER; ERCC6) bzw. die DNA-Schäden signalisierenden sensorischen Systeme (ATM u. ATR-Kinasen) oder die homologe Rekombinations-Reparatur (HR; XRC2), auf der Transkriptionsebene runterreguliert werden (log2FC = -1,09). Insbesondere wurden die Hauptproteine des DNA-Dealkylierungs-Signalwegs (APEX1, TET1 oder ALKBH2), welche bei der Triazen-vermittelten DNA-Dealkylierung eine wichtige Rolle spielen, konsistent nach Behandlung mit Verbindung (IV-A3) in beiden Zelllinien herunterreguliert (vgl. Figur 1B). Eine Behandlung mit Verbindung (IV-A3) bzw. Verbindung (III-A) führt zudem zu einer konsistenten Hochregulation von CDKN1A, begleitet von einer Downregulation von CDK-Proteinen und essentiellen DNA-Reparaturenzymen. Verbindung (IV-A3) induziert zudem bestimmte Veränderungen auf der Transkriptomebene, welche eine Rolle bei der Regulation des Zellzyklus, Membrantransports, Drugmetabolismus und der Zelladhäsion spielen. Darüber hinaus verursacht Verbindung (IV-A3) eine umfassende Downregulation von Transkripten, welche bei verschiedenen DNA-Reparatursystemen funktionell eine wichtige Rolle spielen.

Fig. 1A und Fig. 1B zeigen anhand von Waterfall-Plots rein exemplarisch Transkriptionsmuster von mit Verbindung (IV-A3) behandelten UWB1.289 Ovarialkarzinom-Zellen:
Fig. 1A zeigt die differentiell regulierte Genexpression von Genen, die an Zellzyklusregulation, Drug-Membrantransport, Drug-Metabolismus, Zelladhäsion und anderen selektierten Stoffwechselwegen beteiligt sind, in UWB1.289-Zellen nach Behandlung mit Verbindung (IV-A3). Fig. 1B zeigt die differentielle Genexpression von Genen, die an Stoffwechselwegen, welche die DNA-Reparatur regulieren, beteiligt sind, in UWB1.289-Zellen nach Behandlung mit Verbindung (IV-A3).

Aus den oben beschriebenen Transkriptionsanalysen lässt sich ableiten, dass die erfindungsgemäß eingesetzten Verbindungen auf Basis von Diphenylderivaten bzw. Triazenen einen signifikanten Einfluss auf die Genexpression von Genen haben, die zum einen an verschiedenen DNA-Reparaturmechanismen beteiligt sind, darüber hinaus aber auch Genen, welche an der Zellzyklusregulation, dem Drug-Membrantransport, dem Drug-Metabolismus, der Zelladhäsion und anderen selektierten Stoffwechselwegen beteiligt sind. Ohne sich hierbei auf diese Theorie beschränken zu wollen, wird im Rahmen der vorliegenden Erfindung davon ausgegangen, dass insbesondere der Einfluss auf die Expression von Genen, welche die zellulären Reparatursysteme regulieren bzw. koordinieren, gleichzeitig Grundlage für die antivirale Wirksamkeit der erfindungsgemäß eingesetzten Verbindungen auf Basis von Diphenylderivaten bzw. Triazenen ist.

### 2. Untersuchung der antiviralen Wirksamkeit von erfindungsgemäßen Verbindungen

Um die antivirale Wirksamkeit der erfindungsgemäßen Verbindung zu untersuchen, wurden die nachfolgend beschriebenen Versuche durchgeführt.

Die weiteren experimentellen Versuche wurden mit den folgenden Verbindungen durchgeführt:
- Verbindung der obigen Formel (VI-A), nachfolgend auch einfach nur als "Verbindung (VI-A)" oder "(VI-A)" bezeichnet (eingesetzt in Form des Natriumsalzes) 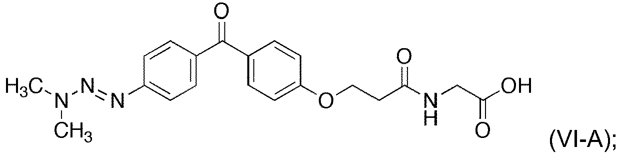
- Verbindung der obigen Formel (VIII-A), nachfolgend auch einfach nur als "Verbindung (VIII-A)" oder "(VIII-A)" bezeichnet 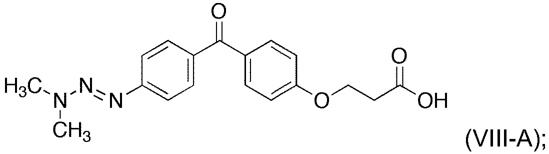
- Verbindung der obigen Formel (IV-A) (erfindungsgemäß), nachfolgend auch einfach nur als "Verbindung (IV-A)" oder "(IV-A)" bezeichnet;
- Verbindung der obigen Formel (IV-B), nachfolgend auch einfach nur als "Verbindung (IV-B)" oder "(IV-B)" bezeichnet

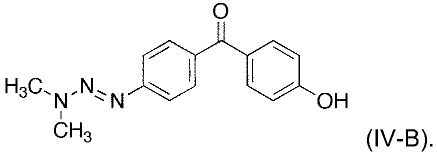

### Untersuchungen des therapeutischen Effekts im Maus-Model

Um den therapeutischen Effekt der Verbindung (VI-A) gegen SARS-CoV-2-Infektionen im Maus-Model zu untersuchen, wurden transgene K18-hACE2-Mäuse eingesetzt. Bei den eingesetzten Viren handelte es sich um ein deutsches Isolat von SARS-CoV-2. Die Studie wurde als Machbarkeitsstudie mit zwei Gruppen (Testgruppe, Kontrollgruppe) auf Basis von jeweils sechs Mäusen, d. h. insgesamt 12 Tieren, durchgeführt. Die beiden Gruppen von Mäusen wurden durch Inhalation von 3 % Isofluran leicht anästhesiert und intranasal mit 300 KWSIU (FFU) von SARS-CoV-2 inokuliert. Die Behandlung mit der Verbindung (VI-A) wurde durch intraperitoneale Injektion bzw. Applikation durchgeführt. Die Behandlung der ersten Gruppe begann 30 Minuten vor der Infektion mit einer Wirkstoffkonzentration bzw. Dosierung von 150 mg/kg. Darüber hinaus wurden den Testmäusen drei Stunden nach der Infektion nochmals 500 mg/kg der Verbindung (gelöst in 100 µl Gesamtvolumen einfach konzentriertem PBS-Puffer mit pH 7,4) verabreicht. In der zweiten Gruppe (Positivkontrolle bzw. Kontrollgruppe) wurden die Mäuse gleichermaßen infiziert, jedoch nur mit PBS-Puffer allein ohne die Zugabe von Diphenylderivaten bzw. Triazenen behandelt (Scheinbehandlung). Zwei Tage post Infektion wurde die Euthanasie der Tiere vorgenommen. Der klinische Wert ("clinical score"), umfassend die Erfassung von Gewicht und klinischer Symptome, der Tiere wurde ermittelt. Um den Therapieerfolg zu ermitteln, wurde die Virenlast in der Lunge mittels qPCR bestimmt. Darüber hinaus wurden immunhistochemische und histopathologische Untersuchungen an der Lunge durchgeführt, nämlich eine Lungenhistologie auf Basis einer Hämatoxylin-Eosin-Färbung und eine Immunofärbung zum Nachweis von SARS-CoV-2-Viren.

Im Rahmen der qPCR hat sich gezeigt, dass die Virenlast in den Lungen der mit der Verbindung (VI-A) behandelten Tiere gegenüber der Positivkontrollgruppe deutlich geringer war. Darüber hinaus haben auch die histologischen Untersuchungen des Lungengewebes ein deutlich reduziertes Infektionsgeschehen gegenüber der Kontrollgruppe gezeigt.

Die Versuche wurden darüber hinaus mit den weiteren Verbindungen (VIII-A), (IV-A) und (IV-B) durchgeführt bzw. wiederholt. Mit diesen Verbindungen wurden gleichermaßen ausgezeichnete Ergebnisse erzielt, welche auf eine deutliche antivirale Wirkung schließen lassen, und zwar aufgrund der erheblich reduzierten Virenlast in der Lunge nach einer Behandlung der Infektion mit SARS-CoV-2 mit den vorgenannten Verbindungen.

### Untersuchung der antiviralen Wirkweise mittels icELISA

Mit Hilfe eines icELISA (in-cell-ELISA) wurde die Menge an viralen SARS-CoV-2 N**-** oder S-Proteinen in infizierten Zellen nach einer Behandlung mit erfindungsgemäßen Verbindungen bestimmt. Für die diesbezüglichen Untersuchungen wurden jeweils die Verbindung (VIII-A), die Verbindung (VI-A), die Verbindung (IV-A) sowie die Verbindung (IV-B) eingesetzt. Für die icELISA-Studien wurden zum einen humane Caco-2-Zellen und zum anderen Vero-E6-Zellen herangezogen.

Der Versuchsaufbau ist nachfolgend geschildert.

### Zellen, Viren und Infektion

Für die ELISA-Untersuchungen wurden Zellen des Typs Vero-E6 (ATCC CRL-1586) und Caco-2 (ATCC HTB-37) zunächst in Dulbecco's Modified Eagle's Medium (DMEM) mit hoher Glukose-Konzentration kultiviert, wobei das Medium mit 10 % fetalem Kälberserum (FCS), Penicillin und Streptomycin supplementiert war. Die Kultivierung erfolgte bei 37 °C bei Atmosphärendruck mit 5 % CO₂. Die SARS-CoV-2-Stämme für die nachfolgende Infektion der Zellen wurden aus Patienten insoliert, wobei die Isolierung und Virenvermehrung in Vero-E6-Zellen erfolgte. Die Isolierung von SARS-CoV-2-Viren wurde diagnostisch mittels qRT-PCR bestätigt. Die Virustiter wurden mittels CCID50 Titration ermittelt.

### Eingesetzte Diphenylderivate bzw. Triazene

Für den nachfolgenden icELISA wurden Lösungen und Verdünnungen der Verbindung (VI-A), der Verbindung (VIII-A), der Verbindung (IV-A) und der Verbindung (IV-B) hergestellt. Die Konzentrationsangaben und die jeweils eingesetzten Lösungsmittel sind der nachfolgenden Tabelle zu entnehmen:

| Verbindung | Stock-lösungen | Medium | 1/4000 | 1/2000 | 1/1000 | 1/500 |
|---|---|---|---|---|---|---|
| (VI-A) | 200 mg/ml | H₂O | 50 µg/ml | 100 µg/ml | 200 µg/ml | 400 µg/ml |
| (VIII-A) | 500 mg/ml | DMSO | 125 µg/ml | 250 µg/ml | 500 µg/ml | 1000 µg/ml |
| (IV-A) | 100 mg/ml | H₂O | 25 µg/ml | 50 µg/ml | 100 µg/ml | 200 µg/ml |
| (IV-B) | 100 mg/ml | DMSO | 25 µg/ml | 50 µg/ml | 100 µg/ml | 200 µg/ml |

Die in der Tabelle spezifizierten Verdünnungen 1/4000, 1/2000, 1/1000 und 1/500 wurden nachfolgend zur Behandlung der Zellen für den icELISA eingesetzt.

### In-cell-ELISA (icELISA)

Zur Quantifizierung der Menge an viralen SARS-CoV-2-Proteinen in infizierten Zellen wurde der icELISA durchgeführt. Für die ELISA-Studien wurden Caco-2-Zellen und Vero-E6-Zellen eingesetzt, wobei je Zelltyp zwei Ansätze gemessen wurden. Der ELISA wurde entsprechend dem in Scholer et al.: "A Novel In-Cell-ELISA Assay Allows Rapid and Automated Quantification of SARS-CoV-2 to Analyze Neutralizing Antibodies and Antiviral Compounds", Front Immunol 11, 573526 (2020), beschriebenen Protokoll durchgeführt. Vor Durchführung des icELISA wurden die Zellen mit SARS-CoV-2 infiziert und 20 Stunden nach der Infektion zur Durchführung des ELISA mit 4 % w/v (Gew./Vol.) Paraformaldehyd in PBS fixiert. Die Zellen wurden mit 1 Vol.-% Triton X-100 in PBS permeabilisiert und mit 3 Vol.-% fetalem Kälberserum in PBS geblockt. Anschließend wurde der erste Antikörper zugegeben und zwei Stunden bei Raumtemperatur bzw. über Nacht bei 4 °C inkubiert. Anschließend wurde der Ansatz mit einem Peroxidase-markierten sekundären Antikörper für ein bis zwei Stunden inkubiert. Anschließend erfolgte ein Waschschritt mit 0,05 Vol.-% Tween-20 in PBS. Um die Enzymreaktion zu visualisieren, wurde Tetramethylbenzidin als Substrat zugefügt. Die Reaktion wurde mit 0,5 M HCl gestoppt, bevor die Absorption mithilfe eines Mikrotiterplatten-Lesegeräts bestimmt und einer geeigneten Software ausgewertet wurde.

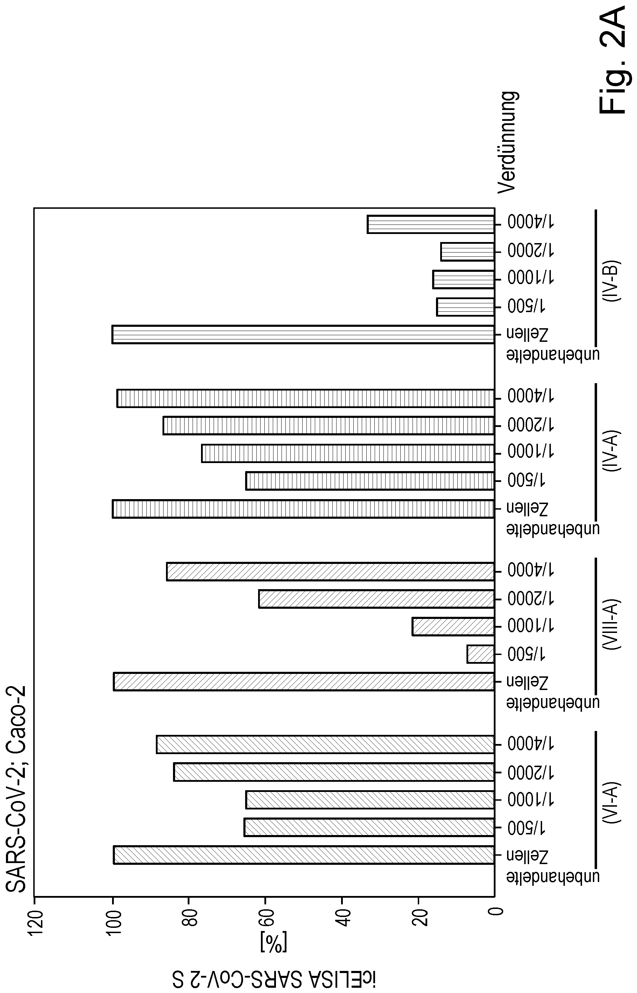

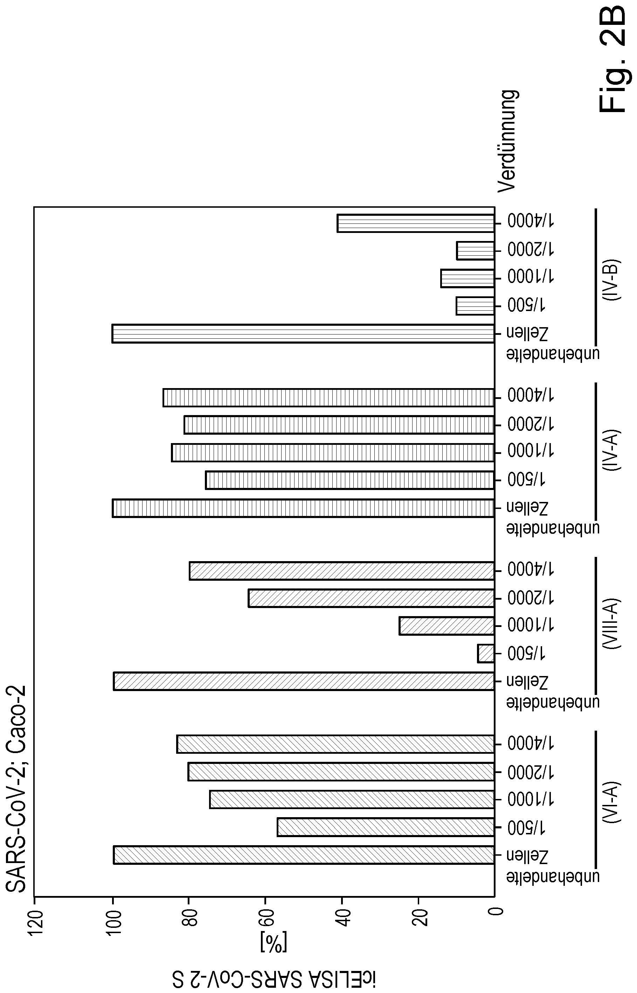

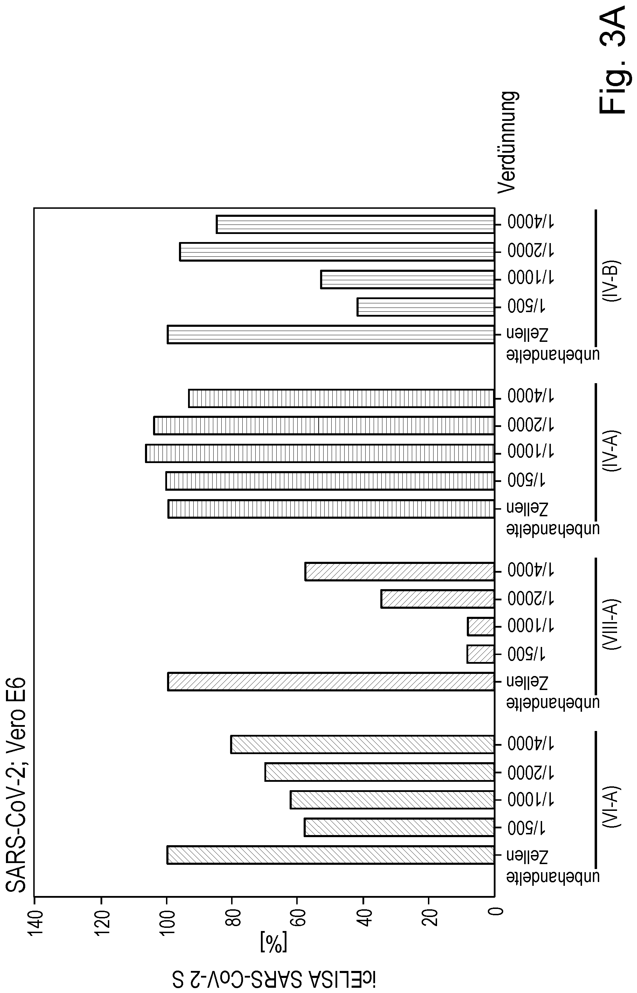

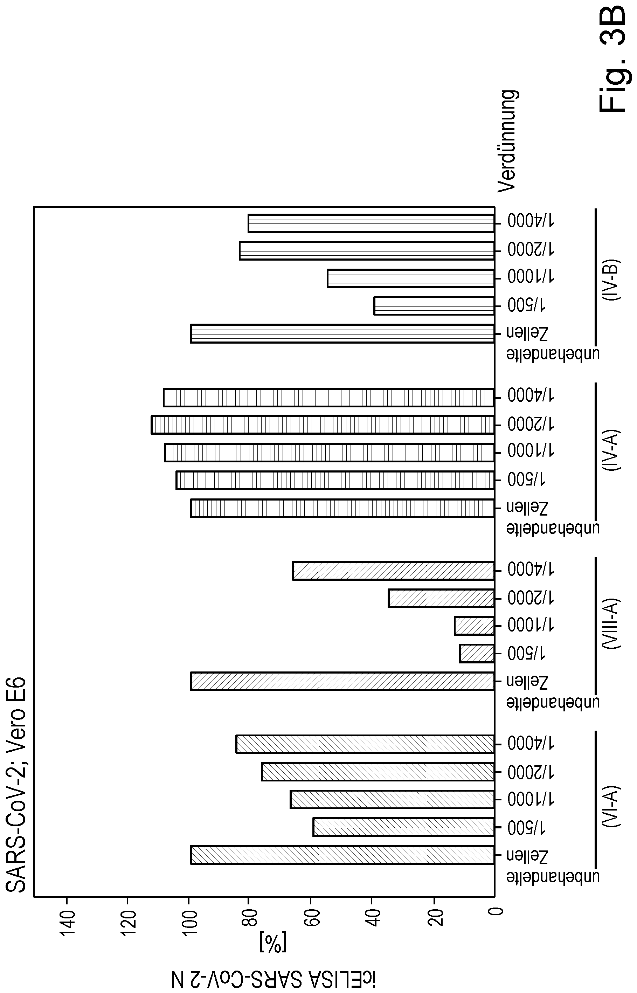

Die für die beiden Ansätze mit Caco-2-Zellen erhaltenen Ergebnisse des icELISA sind in den Figuren 2A und 2B dargestellt. Die Ergebnisse mit Vero-E6-Zellen sind in den Figuren 3A und 3B dargestellt.

Im Rahmen des ELISA wurde die Menge an Viren in den Zellen quantifiziert. Dazu wurde bei der nachfolgenden Auswertung die bei unbehandelten Zellen gemessene Virenlast als Referenzwert mit 100 % festgelegt. Es wurde ermittelt, wie sich die Virenlast prozentual gegenüber dem Referenzwert für unbehandelte Zellen mit den unterschiedlichen Verbindungen in unterschiedlichen Konzentrationen verändert.

In Fig. 2A und 2B sind die Mittelwerte aus jeweils drei unabhängigen Experimenten mit SARS-CoV-2-infizierten Caco-2-Zellen dargestellt. Sowohl aus Fig. 2A als auch Fig. 2B ist ersichtlich, dass mit allen Verbindungen ein signifikanter Rückgang der Viruslast in den behandelten Zellen im Vergleich zu unbehandelten Zellen erzielt werden konnte. Besonders gute Ergebnisse wurden dabei mit den Verbindungen (VIII-A) und (IV-B) erzielt. Bei Verbindung (IV-B) wurde sogar mit der stärksten Verdünnung, d. h. der geringsten Wirkstoffkonzentration, immer noch ein Rückgang der Viruslast um 50 % bzw. 40 % erzielt. Die besten Ergebnisse hinsichtlich des Rückgangs der Viruslast wurden insgesamt mit der Verbindung (VIII-A) in einer Verdünnung von 1/500 (vgl. obige Tabelle) erzielt.

Bei den icELISA-Ergebnissen mit SARS-CoV-2-infizierten Vero-E6-Zellen wurden ähnliche Beobachtungen gemacht (vgl. Fig. 3A und Fig. 3B). Hier wurde ebenfalls der stärkste Rückgang der Viruslast mit der Verbindung (VIII-A) erzielt. Mit der Verbindung (VI-A) und der Verbindung (IV-B) wurde in Vero-E6-Zellen gleichermaßen ein signifikanter Rückgang der Viruslast infolge der Behandlung erzielt.

Die icELISA-Ergebnisse zeigen, dass die erfindungsgemäß eingesetzten Diphenylderivate bzw. Triazene insgesamt zu einem deutlichen Rückgang der SARS-CoV-2-Viruslast in infizierten Zellen führen.

Insgesamt stellen die erfindungsgemäßen Diphenylderivate bzw. Triazene daher einen vielversprechenden Ausgangspunkt für einen Einsatz als Therapeutika zur Behandlung von Viruserkrankungen, insbesondere von durch Corona-Viren ausgelösten Viruserkrankungen, dar.

### Fazit und Ausblick

Vor dem Hintergrund, dass sich die Infektionswege verschiedener Viren bzw. die Mechanismen, mit denen unterschiedliche Viren insbesondere menschlichen Zellen befallen, mitunter ähneln, ist davon auszugehen, dass die erfindungsgemäßen Diphenylderivate bzw. Triazene nicht nur zur Behandlung von Infektionen mit Corona-Viren geeignet sind, sondern auch für die Behandlung von Infektionen mit anderen Virenarten, insbesondere RNA-Viren. Insbesondere erscheinen die erfindungsgemäßen Diphenylderivate bzw. Triazene auch ein geeigneter Ausgangspunkt für die Behandlung von Infektionen mit HI-Viren zu sein. Eine erste Wirksamkeit der erfindungsgemäßen Verbindungen (VI-A), (VIII-A), (IV-A) und (IV-B) wurde mittels EASY-HIT assay festgestellt. Hier konnte bereits eine erste inhibitorische Wirkung auf HI-Viren festgestellt werden.

## Patentansprüche

1. Diphenylderivat zur Verwendung bei der prophylaktischen und/oder therapeutischen Behandlung von Viruserkrankungen, insbesondere Corona-Infektionen, insbesondere COVID-19,
wobei das Diphenylderivat ein Diphenylderivat gemäß der nachstehenden Formel (IV-A1) oder (IV-A2) ist: 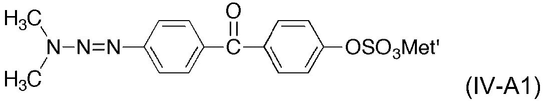 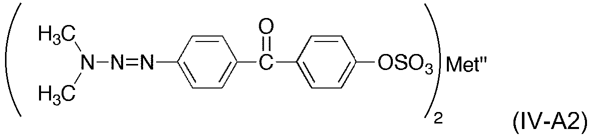
worin:
Met' ein einwertiges Metallion, insbesondere ein Alkalimetallion, vorzugsweise ein Natriumion, bezeichnet und
Met" ein zweiwertiges Metallion, insbesondere ein Erdalkalimetallion, bezeichnet;
oder
wobei das Diphenylderivat ein Diphenylderivat gemäß der nachstehenden Formel (IV-A3) ist: 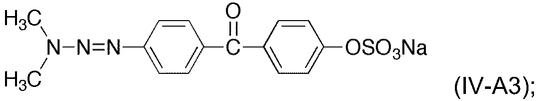 oder
wobei das Diphenylderivat ein Diphenylderivat gemäß der nachstehenden Formel (IV-A) sowie dessen Salze ist: 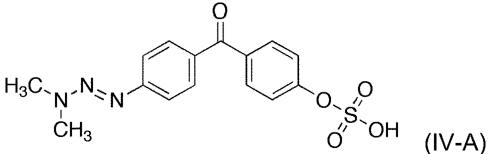 oder
wobei das Diphenylderivat ein Diphenylderivat gemäß den nachstehenden Formeln (IV-B) sowie dessen Salze ist: 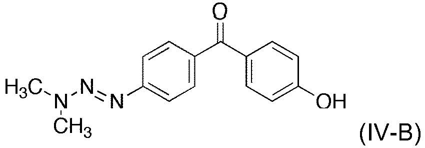 oder
wobei das Diphenylderivat ein Diphenylderivat gemäß der nachstehenden Formel (VI-A) sowie dessen Salze ist: 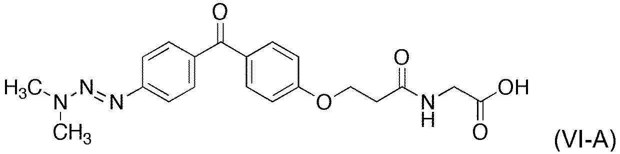 oder
wobei das Diphenylderivat ein Diphenylderivat gemäß der nachstehenden Formel (VIII-A) sowie dessen Salze ist:

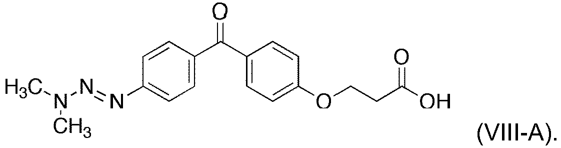

2. Arzneimittel oder Medikament zur Verwendung bei der prophylaktischen und/oder therapeutischen Behandlung von Viruserkrankungen, insbesondere Corona-Infektionen, insbesondere COVID-19, insbesondere Antivirusmittel, enthaltend mindestens ein Diphenylderivat, wie in Anspruch 1 definiert, zusammen mit einem pharmazeutisch verträglichen Träger oder Exzipienten.

3. Diphenylderivat zur Verwendung nach Anspruch 1 oder Arzneimittel oder Medikament zur Verwendung nach Anspruch 2,
wobei das Diphenylderivat, insbesondere das Arzneimittel oder Medikament, systemisch appliziert wird und/oder
wobei das Diphenylderivat, insbesondere das Arzneimittel oder Medikament, zusammen mit mindestens einem weiteren Wirkstoff angewendet oder appliziert wird, insbesondere ausgewählt aus entzündungshemmenden Wirkstoffen, insbesondere Kortikosteroiden, blutverdünnenden Wirkstoffen, antiviral wirkenden Wirkstoffen sowie deren Kombinationen.

4. Pharmazeutische Kombination zur Verwendung bei der prophylaktischen und/oder therapeutischen Behandlung von Viruserkrankungen, insbesondere Corona-Infektionen, insbesondere COVID-19, umfassend
(A) mindestens ein Diphenylderivat, wie in Anspruch 1 definiert; sowie
(B) mindestens einen weiteren Wirkstoff, ausgewählt aus entzündungshemmenden Wirkstoffen, insbesondere Kortikosteroiden, blutverdünnenden Wirkstoffen, antiviral wirkenden Wirkstoffen sowie deren Kombinationen.

5. Pharmazeutische Kombination zur Verwendung nach Anspruch 4, wobei das Diphenylderivat, insbesondere das Arzneimittel oder Medikament, systemisch appliziert wird.

6. Diphenylderivat zur Verwendung nach Anspruch 1, Arzneimittel oder Medikament zur Verwendung nach Anspruch 2 oder pharmazeutische Kombination zur Verwendung nach Anspruch 4 oder 5,
wobei die Viruserkrankungen ausgewählt sind aus der Gruppe von Corona-Infektionen, wie MERS und SARS, insbesondere COVID-19, HIV-Infektionen, Influenza, Parainfluenza, Cytomegalievirus-Infektionen (HCMV-Infektionen), Papillomavirus-Infektionen (HPV-Infektionen), viralen Hepatitiden (Virus-Hepatitiden), Enterovirus-Infektionen, Epstein-Barr-Virus-Infektionen, Herpesviren-Infektionen, Varizellavirus-Infektionen, insbesondere Varizella-zoster-Virus-Infektionen, Adenovirus-Infektionen, Rotavirus-Infektionen, Gelbfiebervirus-Infektionen, viralem hämorrhagischem Fieber (VHF), FSME-Virus-Infektionen, West-Nil-Virus-Infektionen, Ebolavirus-Infektionen, Tollwut (Rabies), Marburgvirus-Infektionen, Hantavirus-Infektionen, Lassa-Virus-Infektionen, Dengue-Virus-Infektionen, Arena-Virus-Infektionen, Arbovirus-Infektionen, Coxsackie-Virus-Infektionen, Masern-, Mumps, Röteln- und Ringelröteln-Virusinfektionen sowie Poliovirus-Infektionen, vorzugsweise aus der Gruppe von Corona-Infektionen, insbesondere COVID-19, HIV-Infektionen, Influenza, Parainfluenza, Cytomegalievirus-Infektionen (HCMV-Infektionen), Papillomavirus-Infektionen (HPV-Infektionen) und viralen Hepatitiden (Virus-Hepatitiden), besonders bevorzugt aus der Gruppe von Corona-Infektionen, insbesondere COVID-19

## Claims

1. Diphenyl derivative for use in the prophylactic and/or therapeutic treatment of viral diseases, in particular corona infections, especially COVID-19,
wherein the diphenyl derivative is a diphenyl derivative according to formula (IV-A1) or (IV-A2) below: 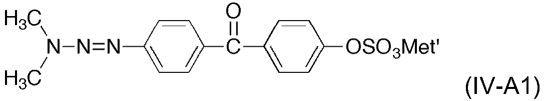 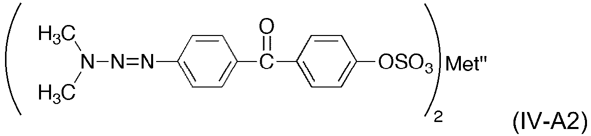
wherein:
Met' denotes a monovalent metal ion, in particular an alkali metal ion, preferably a sodium ion, and
Met" denotes a divalent metal ion, in particular an alkaline earth metal ion;
or
wherein the diphenyl derivative is a diphenyl derivative according to the following formula (IV-A3): 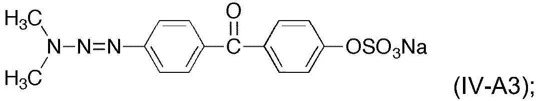 or
wherein the diphenyl derivative is a diphenyl derivative according to formula (IV-A) below and salts thereof: 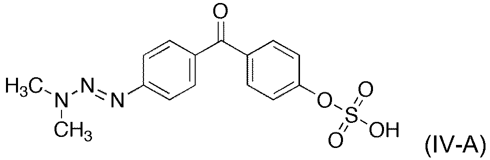 or
wherein the diphenyl derivative is a diphenyl derivative according to the following formulae (IV-B) and salts thereof: 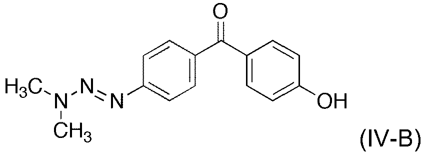 or
wherein the diphenyl derivative is a diphenyl derivative according to the following formula (VI-A) and salts thereof: 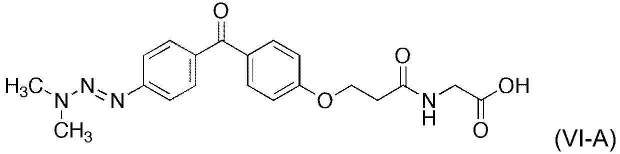 or
wherein the diphenyl derivative is a diphenyl derivative according to the following formula (VIII-A) and salts thereof:

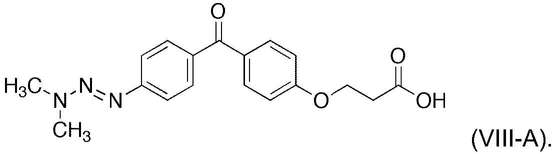

2. A drug or medicament for use in the prophylactic and/or therapeutic treatment of viral diseases, in particular corona infections, in particular COVID 19, in particular antiviral agents, comprising at least one diphenyl derivative as defined in claim 1, together with a pharmaceutically acceptable carrier or excipient.

3. A diphenyl derivative for use according to claim 1 or a drug or medicament for use according to claim 2,
wherein the diphenyl derivative, in particular the drug or medicament, is applied systemically and/or
wherein the diphenyl derivative, in particular the drug or medicament, is used or applied together with at least one further active ingredient, in particular selected from anti-inflammatory active ingredients, in particular corticosteroids, blood-thinning active ingredients, antiviral active ingredients and combinations thereof.

4. Pharmaceutical combination for use in the prophylactic and/or therapeutic treatment of viral diseases, in particular corona infections, in particular COVID-19, comprising
(A) at least one diphenyl derivative as defined in claim 1; and
(B) at least one further active ingredient selected from anti-inflammatory active ingredients, in particular corticosteroids, blood-thinning active ingredients, antiviral active ingredients and combinations thereof.

5. Pharmaceutical combination for use according to claim 4,
wherein the diphenyl derivative, in particular the drug or medication, is applied systemically.

6. Diphenyl derivative for use according to claim 1, drug or medicament for use according to claim 2 or pharmaceutical combination for use according to claim 4 or 5,
wherein the viral diseases are selected from the group of corona infections, such as MERS and SARS, in particular COVID-19, HIV infections, influenza, parainfluenza, cytomegalovirus infections (HCMV infections), papillomavirus infections (HPV infections), viral hepatitis (viral hepatitis), enterovirus infections, Epstein-Barr virus infections, herpes virus infections, varicella virus infections, in particular varicella zoster virus infections, adenovirus infections, rotavirus infections, yellow fever virus infections, viral haemorrhagic fever (VHF), TBE virus infections, West Nile virus infections, Ebola virus infections, rabies, Marburg virus infections, hantavirus infections, Lassa virus infections, dengue virus infections, arena virus infections, arbovirus infections, Coxsackie virus infections, measles, mumps, rubella and rubella virus infections and poliovirus infections, preferably from the group of corona infections, in particular COVID-19, HIV infections, influenza, parainfluenza, cytomegalovirus infections (HCMV infections), papillomavirus infections (HPV infections) and viral hepatitis (viral hepatitis), particularly preferably from the group of corona infections, in particular COVID-19.

## Revendications

1. Dérivé de diphényle destiné à être utilisé dans le traitement prophylactique et/ou thérapeutique de maladies virales, en particulier d'infections à coronavirus, notamment COVID-19,
où le dérivé de diphényle est un dérivé de diphényle représenté par la formule (IV-A1) ou (IV-A2) ci-dessous : 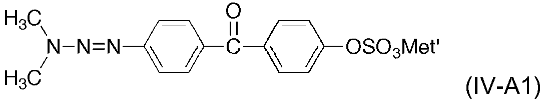 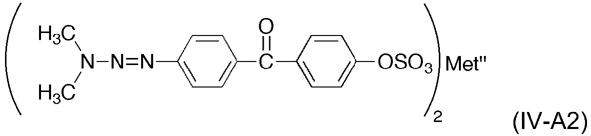 où
Met' désigne un ion métallique monovalent, en particulier un ion de métal alcalin, de préférence un ion sodium, et
Met" désigne un ion métallique divalent, en particulier un ion de métal alcalino-terreux ;
ou
où le dérivé de diphényle est un dérivé de diphényle représenté par la formule (IV-A3) ci-dessous : 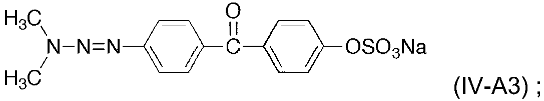 ou
où le dérivé de diphényle est un dérivé de diphényle représenté par la formule (IV-A) ci-dessous, ainsi que ses sels : 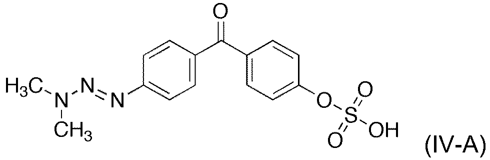 ou
où e dérivé de diphényle est un dérivé de diphényle selon les formules (IV-B) ci-dessous, ainsi que ses sels : 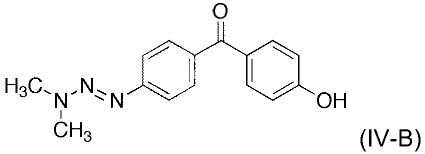 ou
où le dérivé de diphényle est un dérivé de diphényle représenté par la formule (VI-A) ci-dessous, ainsi que ses sels : 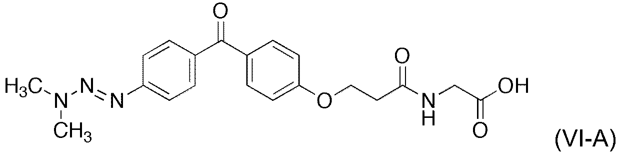 ou
où le dérivé de diphényle est un dérivé de diphényle représenté par la formule (VIII-A) ci-dessous, ainsi que ses sels :

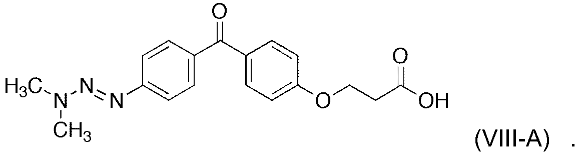

2. Médicament ou produit médicamenteux (pharmaceutique) destiné à être utilisé dans le traitement prophylactique et/ou thérapeutique de maladies virales, en particulier d'infections corona, en particulier COVID-19, notamment agent antiviral, contenant au moins un dérivé de diphényle tel que défini dans la revendication 1, conjointement avec un support ou excipient pharmaceutiquement acceptable.

3. Dérivé de diphényle destiné à utiliser selon la revendication 1 ou médicament ou produit médicamenteux destiné à utiliser selon la revendication 2,
le dérivé de diphényle, en particulier le médicament ou le produit médicamenteux, étant appliqué par voie systémique et/ou
le dérivé de diphényle, en particulier le médicament ou le produit médicamenteux, étant utilisé ou appliqué conjointement avec au moins un autre principe actif, en particulier choisi parmi les principes actifs anti-inflammatoires, en particulier les corticostéroïdes, les principes actifs fluidifiant le sang, les principes actifs à action antivirale ainsi que leurs combinaisons.

4. Combinaison pharmaceutique destinée à être utilisée dans le traitement prophylactique et/ou thérapeutique de maladies virales, en particulier d'infections à coronavirus, notamment COVID-19, comprenant
(A) au moins un dérivé de diphényle tel que défini dans la revendication 1; et
(B) au moins une autre substance active, choisie parmi les substances actives anti-inflammatoires, en particulier les corticostéroïdes, les substances actives fluidifiant le sang, les substances actives à action antivirale ainsi que leurs combinaisons.

5. Combinaison pharmaceutique destinée à être utilisée selon la revendication 4,
le dérivé de diphényle, en particulier le médicament ou le produit médicamenteux, étant appliqué par voie systémique.

6. Dérivé de diphényle destiné à être utilisée selon la revendication 1, médicament ou produit médicamenteux destiné à être utilisée selon la revendication 2 ou combinaison pharmaceutique destinée à être utilisée selon la revendication 4 ou 5,
les maladies virales étant choisies dans le groupe des infections à coronavirus, telles que le MERS et le SRAS, en particulier le COVID-19, des infections à VIH, de la grippe, de la parainfluenza, des infections à cytomégalovirus (infections HCMV), des infections à papillomavirus (infections HPV), des hépatites virales (hépatites virales), infections à entérovirus, infections à virus Epstein-Barr, infections à herpès virus, infections à virus de la varicelle, en particulier les infections à virus varicelle-zona, infections à adénovirus, infections à rotavirus, infections à virus de la fièvre jaune, fièvre hémorragique virale (FHV), infections à virus de la TBE, infections à virus du Nil occidental, infections à virus Ebola, rage (Rabies), infections à virus Marburg, infections à hantavirus, infections à virus Lassa, infections à virus de la dengue, infections à virus Arena, infections à arbovirus, infections à virus Coxsackie, infections à virus de la rougeole, des oreillons, de la rubéole et de l'érythème fessier et infections à poliovirus, de préférence dans le groupe des infections à coronavirus, notamment COVID-19, des infections à VIH, de la grippe, de la parainfluenza, des infections à cytomégalovirus (HCMV), des infections à papillomavirus (HPV) et des hépatites virales (hépatites virales), de préférence dans le groupe des infections à coronavirus, notamment COVID-19.
